# EUROPEAN PATENT APPLICATION

(11) **EP 3 842 426 A1**
(43) Date of publication of application: **30.06.2021**
(21) Application number: 19852424.1
(22) Date of filing: 21.08.2019
(51) Int. Cl.: C07D 403/04, C07D 207/33, C07D 237/00

(54) **PREPARATION METHOD OF PYRROLO-AMINO-PYRIDAZINONE COMPOUND AND INTERMEDIATE THEREOF**

(30) Priority: 22.08.2018 CN 201810958690
(71) Applicant: Jiangsu Hengrui Medicine Co., Ltd., Lianyungang, Jiangsu 222047 (CN); Shanghai Hengrui Pharmaceutical Co., Ltd, Shanghai 200245 (CN)
(72) Inventor: SHAO, Qiyun, Shanghai 200245 (CN); XU, Chao, Shanghai 200245 (CN); LU, Weidong, Shanghai 200245 (CN); FENG, Jun, Shanghai 200245 (CN); SUN, Lichao, Lianyungang, Jiangsu 222047 (CN); QIU, Zhenjun, Lianyungang, Jiangsu 222047 (CN)
(74) Representative: dompatent von Kreisler Selting Werner - Partnerschaft von Patent- und Rechtsanwälten mbB
(86) International application number: PCT/CN2019/101790
(87) International publication number: WO 2020/038405

(57) **Abstract**

Provided are a preparation method of pyrrolo-amino-pyridazinone compound and an intermediate thereof. The reaction conditions are easy to control, the processing following the reaction is simple, the production rate is high, and the method is advantageous for industrial production.

## Description

This application claims the priority of Chinese patent application CN201810958690.X, filed on August 22, 2018. The contents of the Chinese patent application are incorporated herein by reference in their entireties.

### Technical Field

The present disclosure relates to a method for preparing a pyrrolo-amino-pyridazinone compound and an intermediate thereof.

### Background

Immune cells can generally be divided into two types: T cells and B cells, the main function of B cells is to secrete various antibodies to help the body resist various foreign invasion. Bruton's tyrosine protein kinase (BTK) is a member of tyrosine protein kinase subfamily and belongs to the Tec kinases family, which is mainly expressed in B cells and distributed in the lymphatic system, hematopoietic and blood system. B cell receptor (BCR) plays an important role in regulating the proliferation and survival of various lymphomas, including subtypes of chronic lymphocytic leukemia (CLL) and non-Hodgkin's lymphoma (NHL), mantle cell lymphoma (MCL), and diffuse large B cell lymphoma (DLBCL). In addition, it is clinically confirmed that B cells play a role in the pathogenesis of rheumatoid arthritis, systemic lupus erythematosus, multiple sclerosis, and other immune diseases. Bruton's tyrosine protein kinase (BTK) is a key protein kinase in the BCR signaling pathway. It can regulate the maturation and differentiation of normal B cells and is also closely related to a variety of B cell lymphoid tissue disorders. Therefore, the targeted small molecule inhibitor BTK can provide benefits for the treatment of B cell malignancies and autoimmune diseases.

WO2016007185A1 relates to a compound of formula (Ia), namely (*R*)-4-amino-1-(1-(but-2-ynoyl)pyrrolidin-3-yl)-3-(4-(2,6-difluorophenoxy)phenyl)-1,6-dihydro-7*H-*pyrrolo[2,3-d]pyridazin-7-one, this compound is a novel BTK kinase inhibitor, which has improved kinases selectivity, clinical efficacy, indications, and safety, and its structure is as follows:

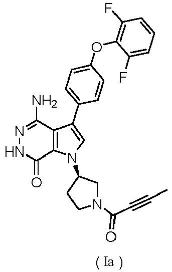

Example 1, intermediate 2 and Example 93 of WO2016007185A1 have disclosed the preparation method of the compound, a total of ten steps of reactions, and the specific reactions are as follows:

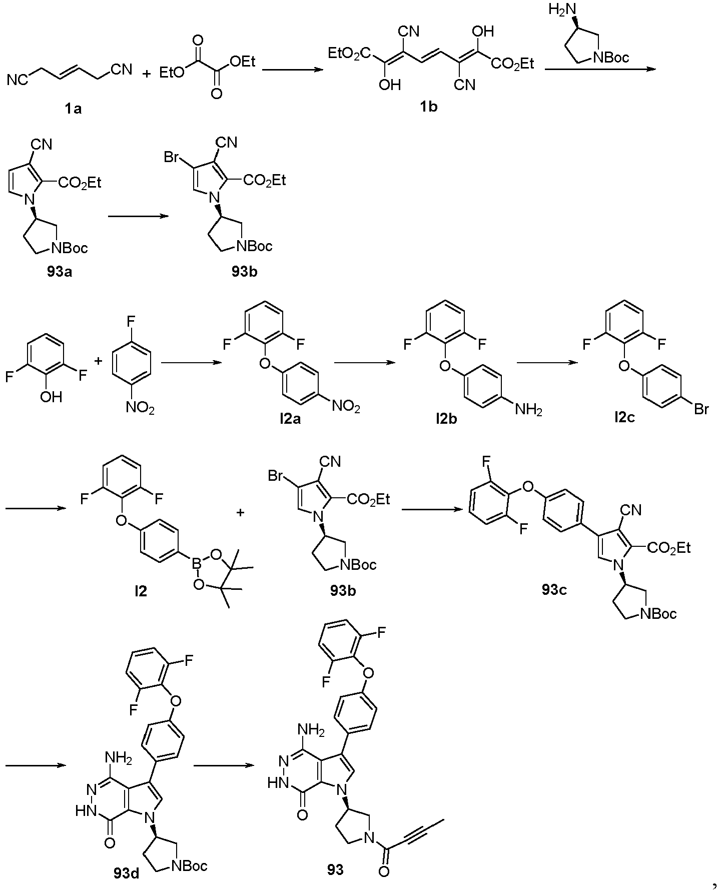

The yield of this method in the preparation of 93c compound is only 22.8%, and the yield of product 93 is only 51%. In the whole method, the yield of multiple reaction steps is low and the purification is difficult, making the total yield of this route low and the feasibility of scale-up poor, and palladium catalyst is used in the method and the cost is high. Therefore, it is necessary to improve the existing preparation method.

### Content of the present invention

The technical problem to be solved by the present disclosure is to provide a method for preparing pyrrolo-amino-pyridazinone compound that is different from the prior art, and the preparation method is optimized by changing the starting materials and intermediates to prepare the target product, in which the starting materials and other reactants are simple and easy to be purchased, the reaction conditions are also simple and controllable with simple post-treatment method, and other ways, to improve the yield and facilitate the industrial expansion of production.

The technical solutions of the present disclosure are as follows.

The present disclosure provides a compound of formula (b), a salt thereof or a stereoisomer thereof, 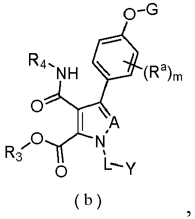 wherein,
A is selected from CR⁰ or N;
R⁰ is selected from hydrogen atom, cyano, carboxyl, hydroxyl, amino, halogen or alkyl;
R^{a} is selected from hydrogen atom, halogen, hydroxyl, nitro, cyano, carboxyl, amino, alkyl, haloalkyl, haloalkoxyl or alkoxyl;
each of R₃, R₄ is independently selected from hydrogen atom, alkyl, alkylcarbonyl, alkoxylcarbonyl, alkylaminocarbonyl, alkylsulfonyl, cycloalkyl, heterocyclyl, aryl or heteroaryl;
G is selected from optionally substituted aryl, heteroaryl, cycloalkyl or heterocyclyl, the substituent is selected from hydrogen atom, halogen, hydroxyl, nitro, cyano, carboxyl, amino, alkyl, alkoxyl, alkylamino, hydroxylalkyl, dialkylamino, alkylcarbonyl, aldehyde alkyl, alkoxycarbonyl, aldehyde alkoxyl, alkylcarbonylamino, alkylaminocarbonyl, alkylsulfonyl, alkenyl, alkenylcarbonyl, alkynyl or alkynylcarbonyl;
L is selected from alkylene or absent;
Y is selected from optionally substituted cycloalkyl, heterocyclyl, aryl or heteroaryl, the substituent is selected from halogen, cyano, alkylcarbonyl, alkoxylcarbonyl, alkylcarbonylamino, alkylsulfonyl, alkylsulfonylamino, alkyl, cycloalkyl, alkenyl, alkenylcarbonyl, alkynyl or alkynylcarbonyl; Y is preferably optionally substituted 3-8 membered heterocyclyl, more preferably optionally substituted pyrrolidinyl or optionally substituted piperidinyl;
m=0, 1, 2 or 3.

In some embodiments, in the compound of formula (b), the salt thereof or the stereoisomer thereof, the A is preferably CR⁰.

In some embodiments, in the compound of formula (b), the salt thereof or the stereoisomer thereof, the R⁰ is preferably hydrogen atom.

In some embodiments, in the compound of formula (b), the salt thereof or the stereoisomer thereof, the R^{a} is preferably hydrogen atom.

In some embodiments, in the compound of formula (b), the salt thereof or the stereoisomer thereof, the R₃ is preferably alkyl or hydrogen.

In some embodiments, in the compound of formula (b), the salt thereof or the stereoisomer thereof, the R₄ is preferably alkyl.

In some embodiments, in the compound of formula (b), the salt thereof or the stereoisomer thereof, the G is preferably substituted aryl, the substituent is preferably halogen.

In some embodiments, in the compound of formula (b), the salt thereof or the stereoisomer thereof, the L is preferably absent.

In some embodiments, in the compound of formula (b), the salt thereof or the stereoisomer thereof, the Y is preferably substituted heterocyclyl, the substituent is preferably alkoxylcarbonyl; the Y is more preferably substituted 3-8 membered heterocyclyl, further preferably substituted pyrrolidinyl or substituted piperidinyl, the most preferably

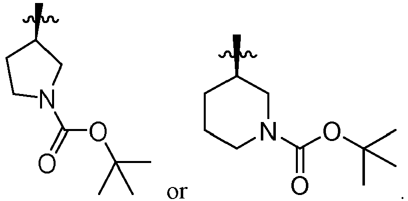

In some embodiments, in the compound of formula (b), the salt thereof or the stereoisomer thereof, the A is CR⁰; the R^{0 is} hydrogen atom; the R^{a} is hydrogen atom; the R₃ is alkyl; the R₄ is alkyl; the G is substituted aryl, the substituent is halogen; the L is absent; the Y is substituted heterocyclyl, the substituent is alkoxylcarbonyl; the Y is more preferably substituted 3-8 membered heterocyclyl, further preferably substituted pyrrolidinyl or substituted piperidinyl, the most preferably

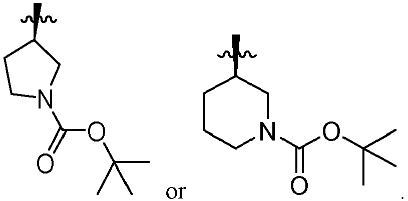

In some embodiments, when L is absent, and Y is connected to other parts of the molecule through the carbon atom on Y, then the carbon atom is in the R configuration.

In some embodiments, the compound of formula (b) described in the above embodiments is selected from

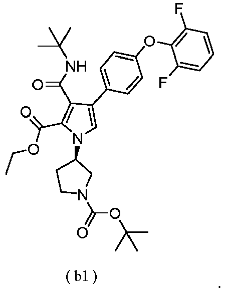

The present disclosure further provides a method for preparing a compound of formula (b) or a stereoisomer thereof, the method comprises 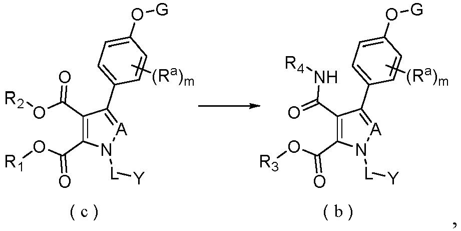 wherein,
in formula (c), each of R₁, R₂ is independently selected from hydrogen atom, alkyl, haloalkyl, benzyl, allyl, trimethylsilyl, triethylsilyl, tetrahydropyranyl or fluorenylmethyl, or R₁ and R₂ combine with the groups they are attached to form a 5-membered cyclic anhydride;
A, R^{a}, R₃, R₄, G, L, Y and m are as defined in formula (b).

In some embodiments, in the compound of formula (c), each of R₁, R₂ is preferably independently selected from hydrogen atom or alkyl, or R₁ and R₂ combine with the groups they are attached to form 5-membered cyclic anhydride; A, R^{a}, G, L, Y and m are as defined in formula (b).

In the above embodiments, the method for preparing the compound of formula (b) or the stereoisomer thereof can also comprise:

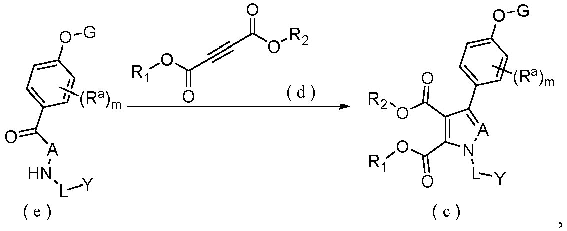

wherein, A, R^{a}, G, L, Y and m are as defined in formula (b); R₁ and R₂ are as defined in formula (c) above.

In the present disclosure, the compound of formula (c) is prepared from the compound of formula (e) (simplified as: formula (e) → formula (c)), the compound of formula (b) is prepared from the compound of formula (c) (simplified as: formula (c) → formula (b)), although this is shown as a one-step reaction, it can be either a one-step or multi-step reaction step, depending on the definitions of the substituents R₁ and R₂:

When R₁ in formula (c-1) is H, then the structure is as shown in formula (c-1), R₂ and R₃ are as defined above but not H, and R₁ in formula (d) and formula (c) is as defined above but not H, the compound of formula (c-1) is prepared from the compound of formula (e) (simplified as: formula (e) → formula (c) → formula (c-1)), which is a two-step reaction, the compound of formula (b) is prepared from the compound of formula (c-1) (simplified as: formula (c-1) → formula (c-2) → formula (c-3) → formula (b-1)→ formula (b)), which is a four-step reaction and is as shown below

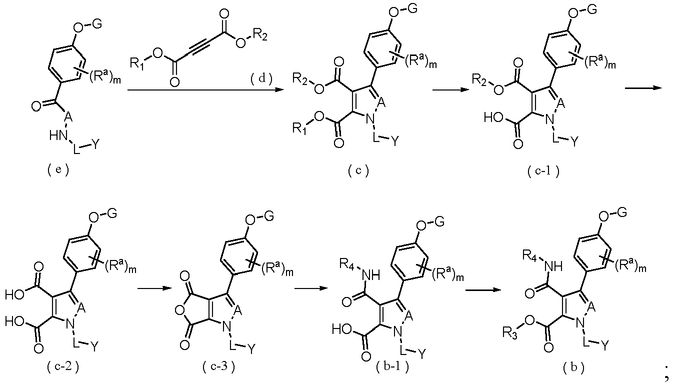

When R₁ is H and R₂ is H in formula (c-2), then the structure is as shown in formula (c-2), and R₂ in formula (d), formula (c-1) and formula (c) is as defined above but not H, R₃ is as defined above but not H, R₁ in formula (d) and formula (c) is as defined above but not H, the compound of formula (c-2) is prepared by formula (e) (simplified as: formula (e) → formula (c) → formula (c-1) → formula (c-2)), which is a three-step reaction, the compound of formula (b) is prepared from formula (c-2) (simplified as: formula (c-2) → Formula (c-3) → Formula (b-1) → Formula (b)), which is a three-step reaction, which is as shown below

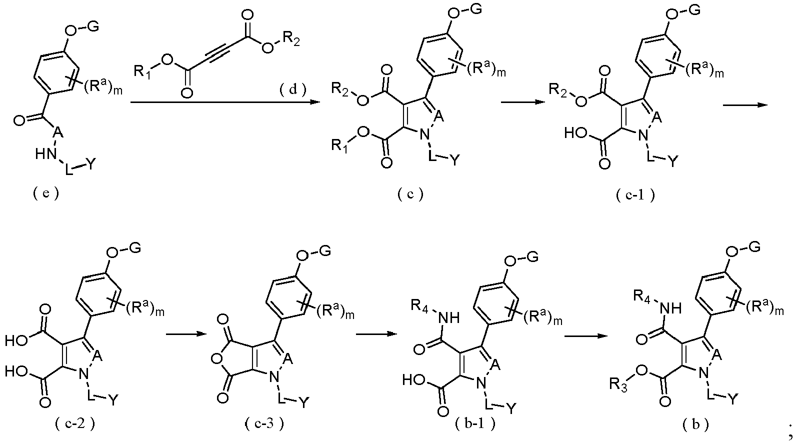

When R₁ and R₂ in formula (c-2) are H at the same time, cyclic anhydride is obtained by dehydration, and the structure is shown in formula (c-3), R₂ in formula (d), formula (c-1) and formula (c) is as defined above but not H, R₃ is as defined above but not H, R₁ in formula (d) and formula (c) is as defined above but not H, and the compound of formula (c-3) is prepared from formula (e) (simplified as: formula (e) →formula (c) →formula (c-1) → formula (c-2) →formula (c-3)), which is a four-step reaction, the compound of formula (b) is prepared from the compound of formula (c-3) (simplified as: formula (c-3) → formula (b-1) → formula (b)), which is a two-step reaction and is shown as follow

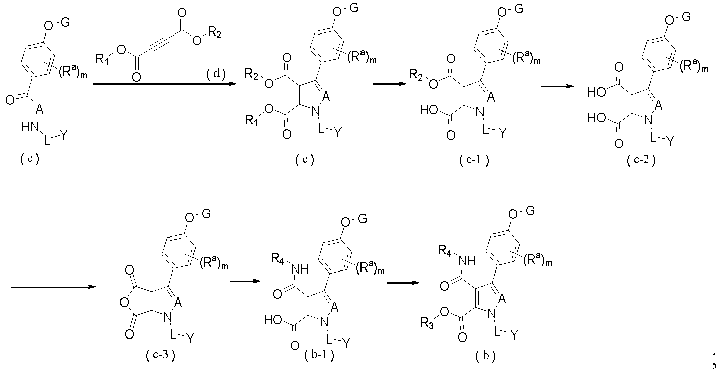

A, R^{a}, R₄, G, L, Y and m are as defined in formula (b).

In the process of preparing the compound of formula (c) from the compound of formula (e), the temperature of the reaction is preferably 70 to110°C; the time of the reaction is preferably 1 to 4 hours; the reaction solvent of the reaction is preferably amides solvent, further preferably is *N*,*N*-dimethylformanmide; the molar concentration of the compound of formula (e) in the solvent is preferably 0.1 to 0.6 mol/L; the molar ratio of the compound of formula (e) to the compound of formula (d) is preferably 1:1 to 1:5.

In the process of preparing the compound of formula (c-1) from the compound of formula (c), the temperature of the reaction is preferably 70 to 110°C; the time of the reaction is preferably 3 to 8 hours; the reaction is preferably carried out in an alkaline solution, the alkali in the alkaline solution is preferably one or more selected from potassium carbonate, sodium carbonate, cesium carbonate, sodium bicarbonate, potassium bicarbonate, sodium hydroxide, potassium hydroxide, lithium hydroxide, sodium acetate, potassium acetate, sodium methoxide, potassium methoxide, triethylamine, *N*,*N*-diisopropylethylamine, ammonia and pyridine, preferably potassium hydroxide; the reaction solvent for the reaction is preferably a mixed solvent of amide solvent and water, the amide solvent is preferably *N,N*-dimethylformanmide; the molar concentration of the compound of formula (c) in the reaction solvent is preferably 0.1 to 0.4 mol/L; the molar ratio of the compound of formula (c) and the alkali is preferably 1:10 to 1:20.

In the process of preparing the compound of formula (c-2) from the compound of formula (c-1), the temperature of the reaction is preferably refluxing the solvent of the reaction; the time of the reaction is preferably 3 to 8 hours; the reaction is preferably carried out in an alkaline solution, the alkali in the alkaline solution is preferably one or more selected from potassium carbonate, sodium carbonate, cesium carbonate, sodium bicarbonate, potassium bicarbonate, sodium hydroxide, potassium hydroxide, lithium hydroxide, sodium acetate, potassium acetate, sodium methoxide, potassium methoxide, triethylamine, *N,N-*diisopropylethylamine, ammonia and pyridine, preferably potassium hydroxide; the reaction solvent is preferably one or more selected from methanol, ethanol, propanol, butanol, ethylene glycol, acetonitrile, propionitrile, succinonitrile, *N*,*N-*dimethylformanmide, *N,N-*dimethylacetamide, acetone, butanone, tetrahydrofuran, dimethyl sulfoxide, sulfolane, N-methylpyrrolidone, formamide, acetamide and water, preferably methanol and water; the molar concentration of the compound of formula (c-1) in the solvent is preferably 0.1 to 0.4 mol/L; the molar ratio of the compound of formula (c-1) to the alkali is preferably 1:3 to 1:15.

In the process of preparing the compound of formula (c-3) from the compound of formula (c-2), the temperature of the reaction is preferably room temperature; the time of the reaction is preferably 3 to 7 hours; the reaction solvent is preferably one or more selected from halogenated hydrocarbon solvent, aromatic hydrocarbon solvent, ketone solvent, ether solvent, aliphatic hydrocarbon solvent, glycol derivative solvent, amide solvent, sulfone solvent and sulfoxide solvent, preferably ether solvent, more preferably tetrahydrofuran; the molar concentration of the compound of formula (c-2) in the solvent is preferably 0.05 to 0.4 mol/L; preferably, the compound of formula (c-3) is obtained through the reaction of the compound of formula (c-2) with acetic anhydride, and the molar ratio of the compound of formula (c-2) to acetic anhydride is preferably 1:5 to 1:30.

In the process of preparing the compound of formula (b-1) from the compound of formula (c-3), the temperature of the reaction is preferably -10°C to 5°C; the time of the reaction is preferably 1 to 4 hours; the reaction solvent is one or more selected from halogenated hydrocarbon solvent, aromatic hydrocarbon solvent and ether solvent, preferably halogenated hydrocarbon solvent, more preferably dichloromethane; the molar concentration of the compound of formula (c-2) in the solvent is preferably 0.05 to 0.4 mol/L; preferably, the compound of formula (b-1) is obtained by the compound of formula (c-3) under the action of a base, and the base is preferably organic base, further preferably *tert*-butylamine; the molar ratio of the compound of formula (c-3) to the base is preferably 1:1.5 to 1:5.

In the process of preparing the compound of formula (b) from the compound of formula (b-1), the temperature of the reaction is preferably refluxing the solvent of the reaction; the time of the reaction is preferably 8 to 13 hours; the reaction is preferably carried out under the action of an alkali, the alkali is preferably one or more selected from potassium carbonate, sodium carbonate, cesium carbonate, sodium bicarbonate, potassium bicarbonate, sodium hydroxide, potassium hydroxide, lithium hydroxide, sodium acetate, potassium acetate, sodium methoxide, potassium methanol, triethylamine, *N*,*N*-diisopropylethylamine, ammonia and pyridine, preferably potassium carbonate; the reaction solvent is preferably one or more selected from halogenated hydrocarbon solvent, aromatic hydrocarbon solvent, ether solvent, ketone solvent, glycol derivative solvent, amide solvent, sulfone solvent, sulfoxide solvent and aliphatic hydrocarbon solvent, preferably ether solvent, more preferably tetrahydrofuran; the molar concentration of the compound of formula (b-1) in the solvent is preferably 0.05 to 0.4 mol/L; the molar ratio of the compound of formula (b-1) to the alkali is preferably 1:3 to 1:8; preferably, the compound of formula (b) is obtained by the reaction of the compound of formula (b-1) with diethyl sulfate, and the molar ratio of the compound of formula (b-1) to diethyl sulfate is preferably 1:1 to 1:3.

When R₁ is H and R₂ is H in formula (C-1'), then the structure is as shown in formula (C-1'), and R₁ and R₂ in formula (d) and formula (C') are as defined above but not H, the compound of formula (C-1') is prepared from the compound of formula (E') (simplified as: formula (E') → formula (C') → formula (C-1')), which is a two-step reaction, the compound of formula (B') is prepared from the compound of formula (C-1') (simplified as: formula (C-1') → formula (C-2') → formula (B')) which is a two-step reaction and is as shown below

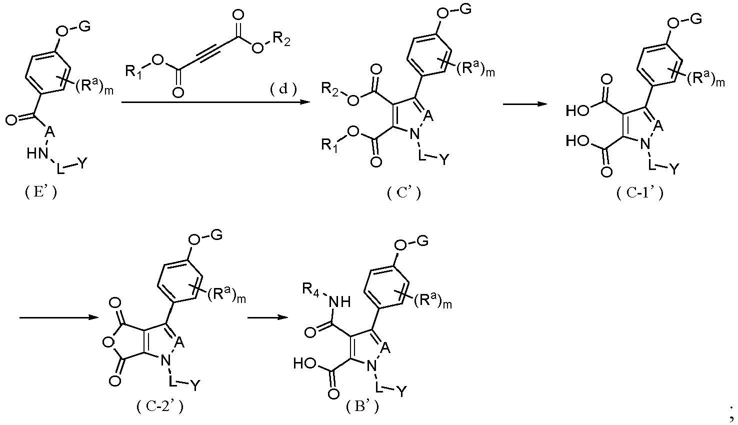

When R₁ and R₂ in formula (C-1') are H at the same time, then a cyclic anhydride is obtained by dehydration, and the structure is as shown in formula (C-2'), when R₁ and R₂ are as defined above but not H, the compound of formula (C-1') is prepared from the compound of formula (E') (simplified as: formula (E') → formula (C') → formula (C-1')), which is a two-step reaction, the compound of formula (B') is prepared from the compound of formula (C-1') (simplified as: formula (C-1') → formula (C-2') → formula (B')), which is a two-step reaction and is as shown below

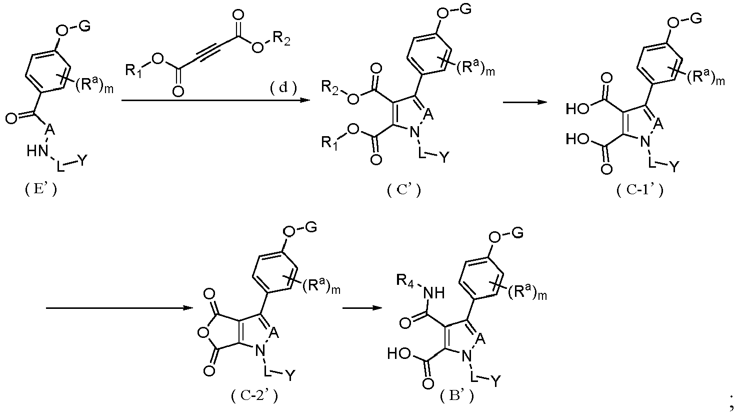

A, R^{a}, R₄, G, L, Y and m are as defined in formula (b).

In the process of preparing the compound of formula (C') from the compound of formula (E'), the temperature of the reaction is preferably refluxing the solvent of the reaction; the time of the reaction is preferably 0.5 to 3 hours (for example, 1.5 hours); the reaction solvent is preferably alcohol solvent, and further preferably methanol; the molar concentration of the compound of formula (E') in the reaction solvent is preferably 0.1 to 0.3 mol/L; the molar ratio of the compound of formula (E') to the compound of formula (d) is preferably 1:1 to 1:5.

In the process of preparing the compound of formula (C-1') from the compound of formula (C'), the temperature of the reaction is preferably refluxing the solvent of the reaction; the time of the reaction is preferably 20 to 35 hours (for example, 28 hours); the reaction is preferably carried out in an alkaline solution, and the alkali in the alkaline solution is preferably one or more selected from potassium carbonate, sodium carbonate, cesium carbonate, sodium bicarbonate, potassium bicarbonate, sodium hydroxide, potassium hydroxide, lithium hydroxide, sodium acetate, potassium acetate, sodium methoxide, potassium methoxide, triethylamine, *N*,*N*-diisopropylethylamine, ammonia and pyridine, preferably potassium hydroxide; preferably, the reaction solvent is one or more selected from a mixed solvent of alcohol solvent and water, the alcohol solvent is preferably methanol; the molar concentration of the compound of formula (C') in the reaction solvent is preferably 0.01 to 0.2 mol/L; the molar ratio of compound of formula (C') to the alkali is preferably 1:10 to 1:30.

In the process of preparing the compound of formula (C-2') from the compound of formula (C-1'), the temperature of the reaction is preferably refluxing the solvent of the reaction; the time of the reaction is preferably 0.5 to 3 hours; the reaction solvent is one or more selected from halogenated hydrocarbon solvent, aromatic hydrocarbon solvent, ether solvent, ketone solvent, glycol derivative solvent, amide solvent, sulfone solvent, sulfoxide solvent and aliphatic hydrocarbon solvent, preferably ether solvent, more preferably tetrahydrofuran; the molar concentration of the compound of formula (C-1') in the solvent is preferably 0.05 to 0.4 mol/L; preferably, the compound of formula (C-2') is obtained by the reaction of the compound of formula (C-2') with acetic anhydride, and the molar ratio of the compound of formula (C-1') to acetic anhydride is preferably 1:0.5 to 1:3.

In the process of preparing the compound of formula (B') from the compound of formula (C-2'), the time of the reaction is preferably 0.5 to 3 hours; the reaction is preferably carried out under the action of a base, and the base is preferably *tert*-butylamine; the reaction solvent is one or more selected from halogenated hydrocarbon solvent, aromatic hydrocarbon solvent, ether solvent, ketone solvent, glycol derivative solvent, amide solvent, sulfone solvent, sulfoxide solvent and aliphatic hydrocarbon solvent, preferably halogenated hydrocarbon solvent, more preferably dichloromethane; the molar concentration of the compound of formula (C-2') in the solvent is preferably 0.05 to 0.4 mol/L; the molar ratio of the compound of formula (C-2') to the base is preferably 1:1 to 1:2.

In the above embodiments, the method for preparing the compound of formula (b) or the stereoisomer thereof can also comprise 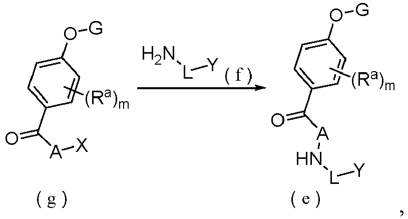 wherein,
A, R^{a}, G, L, Y and m are as defined in formula (b); X in formula (g) is selected from fluorine atom, chlorine atom, bromine atom or iodine atom.

In some embodiments, in the formula (g), X is preferably bromine atom.

In the process of preparing the compound of formula (e) from the compound of formula (g), the temperature of the reaction is preferably 0 to -50°C; the time of the reaction is preferably 1 to 30 hours; the reaction solvent is preferably one or more selected from nitrile solvent, amide solvent, ketone solvent, ether solvent, sulfone solvent and sulfoxide solvent, preferably amide solvent, more preferably *N,N*-dimethylformanmide; the molar concentration of the compound of formula (g) in the solvent is preferably 0.1 to 0.6 mol/L; the molar ratio of the compound of formula (g) to the compound of formula (f) is preferably 1:1 to 1:1.5; the reaction is preferably carried out under the action of a base, the base is preferably organic base, and further preferably *N*,*N*-diisopropylethylamine; the molar ratio of the compound of formula (g) to the base is preferably 1:1 to 1:1.5.

In the above embodiments, the method for preparing the compound of formula (b) or the stereoisomer thereof can also comprise

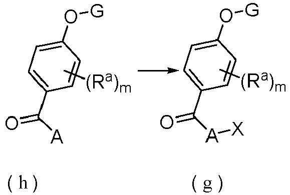

wherein, A, R^{a}, G and m are as defined in formula (b); X is as defined in formula (g) above.

In the process of preparing the compound of formula (g) from the compound of formula (h), the temperature of the reaction is preferably room temperature; the time of the reaction is preferably 15 to 25 hours; the reaction solvent is preferably one or more selected from nitrile solvent, ketone solvent, ether solvent, amide solvent, sulfone solvent and sulfoxide solvent, preferably nitrile solvent, more preferably acetonitrile; the molar concentration of the compound of formula (h) in the solvent is preferably 0.1 to 0.6 mol/L; preferably, the compound of formula (g) is obtained by the reaction of the compound of formula (h) under the action of *N*-bromosuccinimide, and the molar ratio of the compound of formula (h) to *N-*bromosuccinimide is preferably 1:1 to 1:1.5.

In the above embodiments, the method for preparing the compound of formula (b) or the stereoisomer thereof can also comprise 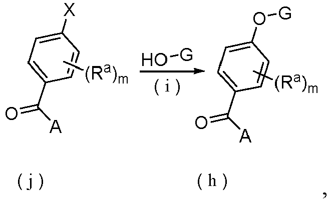 wherein,
A, R^{a}, G and m are as defined in formula (b); X in formula (j) is selected from fluorine atom, chlorine atom, bromine atom or iodine atom.

In some embodiments, X in formula (j) is preferably fluorine atom.

In the process of preparing the compound of formula (h) from the compound of formula (j), the reaction is preferably carried out in an alkaline medium, and the alkaline medium is one or more selected from potassium carbonate, sodium carbonate, cesium carbonate, sodium bicarbonate, potassium bicarbonate, sodium hydroxide, potassium hydroxide, lithium hydroxide, sodium acetate, potassium acetate, sodium methoxide, potassium methoxide, triethylamine, *N*,*N*-diisopropylethylamine, ammonia and pyridine, preferably potassium carbonate; the reaction temperature is preferably 120 to 180°C; the time of the reaction is preferably 20 to 30 hours; the reaction solvent is preferably amide solvent, further preferably dimethylacetamide; the molar concentration of the compound of formula (j) in the solvent is preferably 0.5 to 3 mol/L; the molar ratio of the compound of formula (j) to the compound of formula (i) is preferably 1:1 to 3:1; the molar ratio of the compound of formula (j) to the alkaline medium is preferably 1:1 to 1:5.

The present disclosure also provides a method for preparing a compound of formula (a) or a stereoisomer thereof, comprising 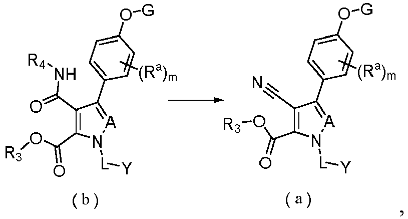 wherein,
R^{a}, R₃, R₄, A, G, L, Y and m are as defined in formula (b), and R₃ is not H;
or, preparing the compound of formula (a) from the compound of formula (b-1), 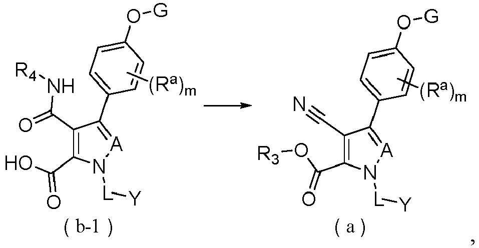 wherein,
   R^{a}, R₃, R₄, A, G, L, Y and m are as defined in formula (b).

In the process of preparing the compound of formula (a) from the compound of formula (b), the temperature of the reaction is preferably room temperature; the time of the reaction is preferably 2 to 7 hours; the reaction solvent is preferably one or more selected from halogenated hydrocarbon solvent, aromatic hydrocarbon solvent and ether solvent, preferably halogenated hydrocarbon solvent, more preferably dichloromethane; the molar concentration of the compound of formula (b) in the solvent is preferably 0.05 to 0.4 mol/L; preferably, the compound of formula (a) is obtained by the reaction of the compound of formula (b) with trifluoroacetic anhydride, and the molar ratio of the compound of formula (b) to trifluoroacetic anhydride is preferably 1:1.5 to 1:3.

In the process of preparing the compound of formula (a) from the compound of formula (b-1), the temperature of the reaction is preferably room temperature; the time of the reaction is preferably 2 to 7 hours; the reaction solvent is preferably one or more selected from halogenated hydrocarbon solvent, aromatic hydrocarbon solvent and ether solvent, preferably halogenated hydrocarbon solvent, more preferably dichloromethane; the molar concentration of the compound of formula (b-1) in the solvent is preferably 0.05 to 0.4 mol/L; preferably, the compound of formula (a) is obtained by the reaction of the compound of formula (b-1) with trifluoroacetic anhydride, and the molar ratio of the compound of formula (b-1) to the trifluoroacetic anhydride is preferably 1:1 to 1:3.

In the above embodiments, the method for preparing the compound of formula (a) or the stereoisomer thereof can also comprise 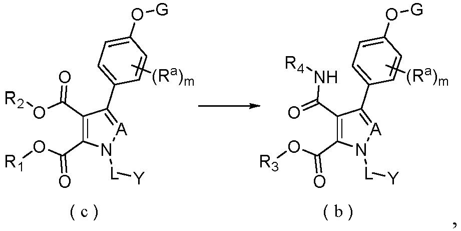 wherein,
each of R₁, R₂ is independently selected from hydrogen atom, alkyl, haloalkyl, benzyl, allyl, trimethylsilyl, triethylsilyl, tetrahydropyranyl or fluorene methyl, or R₁ and R₂ combine with the groups they are attached to form a 5-membered cyclic anhydride;
A, R^{a}, R₃, R₄, G, L, Y and m are as defined in formula (b).

In some embodiments, in the compound of formula (c), each of R₁, R₂ is preferably independently selected from hydrogen atom or alkyl, or R₁ and R₂ combine with the groups they are attached to form a 5-membered cyclic anhydride.

In the above embodiments, the method for preparing the compound of formula (a) or the stereoisomer thereof can also comprise 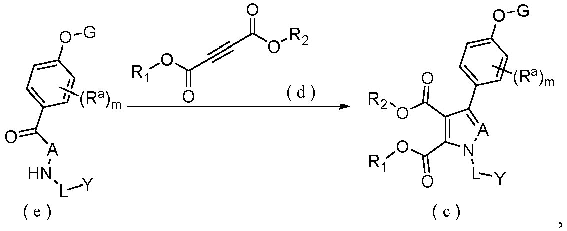 wherein, A, R^{a}, G, L, Y and m are as defined in formula (b); R₁ and R₂ are as defined in formula (c) above.

In the above embodiments, the method for preparing the compound of formula (a) or the stereoisomer thereof can also comprise 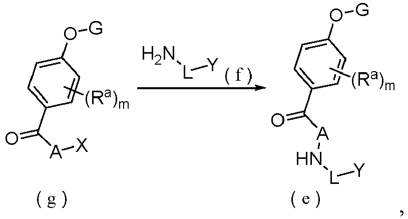 wherein,
A, R^{a}, G, L, Y and m are as defined in formula (b); X is selected from fluorine atom, chlorine atom, bromine atom or iodine atom.

In some embodiments, in the formula (g), X is preferably bromine atom.

In the above embodiments, the method for preparing the compound of formula (a) or the stereoisomer thereof can also comprise 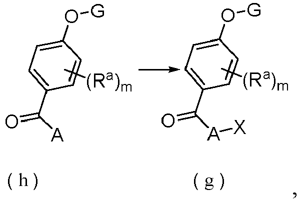 wherein, A, R^{a}, G and m are as defined in formula (b); X is as defined in formula (g) above.

In the above embodiments, the method for preparing the compound of formula (a) or the stereoisomer thereof can also comprise 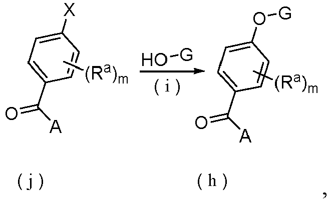 wherein,
A, R^{a}, G and m are as defined in formula (b); X is selected from fluorine atom, chlorine atom, bromine atom or iodine atom.

In some embodiments, X in the formula (j) is preferably fluorine atom.

The present disclosure provides a method for preparing a compound of formula (I) or a stereoisomer thereof, comprising 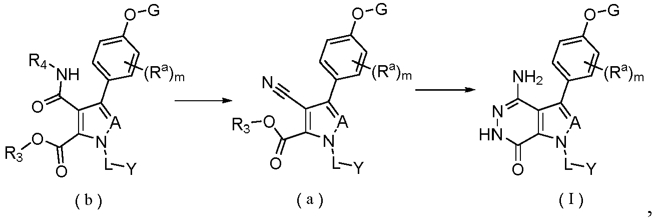 wherein,
A is selected from CR⁰ or N;
R⁰ is selected from hydrogen atom, cyano, carboxyl, hydroxyl, amino, halogen or alkyl;
R^{a} is selected from hydrogen atom, halogen, hydroxyl, nitro, cyano, carboxyl, amino, alkyl, haloalkyl, haloalkoxyl or alkoxyl;
each of R₃, R₄ is independently selected from hydrogen atom, alkyl, alkylcarbonyl, alkoxylcarbonyl, alkylaminocarbonyl, alkylsulfonyl, cycloalkyl, heterocyclyl, aryl or heteroaryl;
G is selected from optionally substituted aryl, heteroaryl, cycloalkyl or heterocyclyl, the substituent is selected from hydrogen atom, halogen, hydroxyl, nitro, cyano, carboxyl, amino, alkyl, alkoxyl, alkylamino, hydroxylalkyl, dialkylamino, alkylcarbonyl, aldehyde alkyl, alkoxycarbonyl, aldehyde alkoxyl, alkylcarbonylamino, alkylaminocarbonyl, alkylsulfonyl, alkenyl, alkenylcarbonyl, alkynyl or alkynylcarbonyl;
L is selected from alkylene or absent;
Y is selected from optionally substituted cycloalkyl, heterocyclyl, aryl or heteroaryl, the substituent is selected from halogen, cyano, alkylcarbonyl, alkoxylcarbonyl, alkylcarbonylamino, alkylsulfonyl, alkylsulfonylamino, alkyl, cycloalkyl, alkenyl, alkenylcarbonyl, alkynyl or alkynylcarbonyl, Y is preferably optionally substituted 3-8 membered heterocyclyl, more preferably optionally substituted pyrrolidinyl or optionally substituted piperidinyl;
m=0, 1, 2 or 3.

In some embodiments, in the method for preparing the compound of formula (I) or the stereoisomer thereof, the A is preferably CR⁰.

In some embodiments, in the method for preparing the compound of formula (I) or the stereoisomer thereof, the R⁰ is preferably hydrogen atom.

In some embodiments, in the method for preparing the compound of formula (I) or the stereoisomer thereof, the R^{a} is preferably hydrogen atom.

In some embodiments, in the method for preparing the compound of formula (I) or the stereoisomer thereof, the R₃ is preferably alkyl or H.

In some embodiments, in the method for preparing the compound of formula (I) or the stereoisomer thereof, the R₄ is preferably alkyl.

In some embodiments, in the method for preparing the compound of formula (I) or the stereoisomer thereof, the G is preferably substituted aryl, the substituent is preferably halogen.

In some embodiments, in the method for preparing the compound of formula (I) or the stereoisomer thereof, the L is preferably absent.

In some embodiments, in the method for preparing the compound of formula (I) or the stereoisomer thereof, the Y is preferably substituted heterocyclyl, the substituent is preferably alkoxylcarbonyl, alkenylcarbonyl or alkynylcarbonyl; the Y is further preferably substituted 3-8 membered heterocyclyl, more preferably substituted pyrrolidinyl or substituted piperidinyl; in formula (b) and (a), the substituent is preferably alkoxylcarbonyl; in formula (I), the substituent is preferably alkenylcarbonyl or alkynylcarbonyl.

In the above embodiments, the method for preparing the compound of formula (I) or the stereoisomer thereof can also comprise 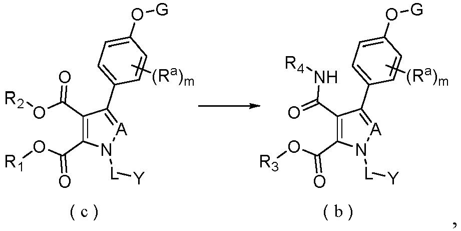 wherein,
each of R₁, R₂ is independently selected from hydrogen atom, alkyl, haloalkyl, benzyl, allyl, trimethylsilyl, triethylsilyl, tetrahydropyranyl or fluorene methyl, or R₁ and R₂ combine with the groups they are attached to form a 5-membered cyclic anhydride;
A, R^{a}, R₃, R₄, G, L, Y and m are as defined in formula (b).

In some embodiments, in the compound of formula (c), each of R₁, R₂ is independently preferably hydrogen atom or alkyl, or R₁ and R₂ combine with the groups they are attached to form 5-membered cyclic anhydride; A, R^{a}, G, L, Y and m are as defined in formula (b).

In the above embodiments, the method for preparing the compound of formula (I) or the stereoisomer thereof can also comprise 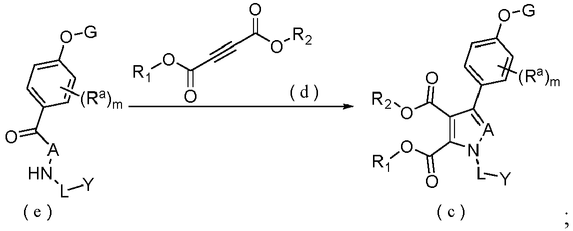 wherein, A, R^{a}, G, L, Y and m are as defined in formula (b); R₁ and R₂ are as defined in formula (c) above.

In the above embodiments, the method for preparing the compound of formula (I) or the stereoisomer thereof can also comprise 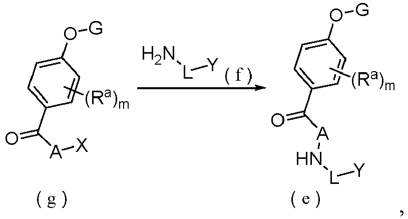 wherein,
A, R^{a}, G, L, Y and m are as defined in formula (b); X is selected from fluorine atom, chlorine atom, bromine atom or iodine atom.

In some embodiments, in the formula (g), X is preferably bromine atom.

In the above embodiments, the method for preparing the compound of formula (I) or the stereoisomer thereof can also comprise 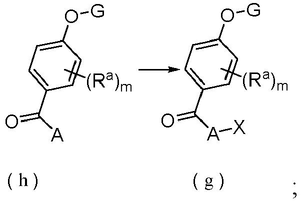 wherein, A, R^{a}, G and m are as defined in formula (b); X is as defined in formula (g) above.

In the above embodiments, the method for preparing the compound of formula (I) or the stereoisomer thereof can also comprise 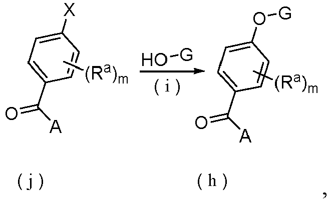 wherein,
A, R^{a}, G and m are as defined in formula (b); X is selected from fluorine atom, chlorine atom, bromine atom or iodine atom.

In some embodiments, X in the formula (j) is preferably fluorine atom.

In the above embodiments, the carbonyl in the compound of formula (I) can also undergo enol interconversion, specifically as shown below 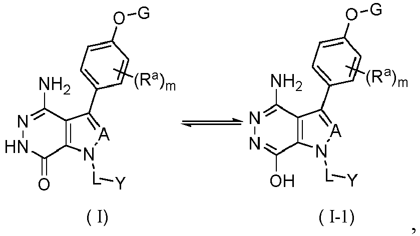 wherein, A, R^{a}, G, L, Y and m are as defined in formula (b).

In some embodiments, the carbonyl in the compound of formula (II') can also undergo enol interconversion, 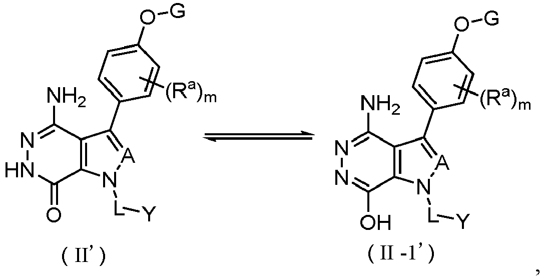 wherein, A, R^{a}, G, L, Y and m are as defined in formula (b).

In some embodiments, the carbonyl in the compound of formula (II) can also undergo enol interconversion, specifically as shown below

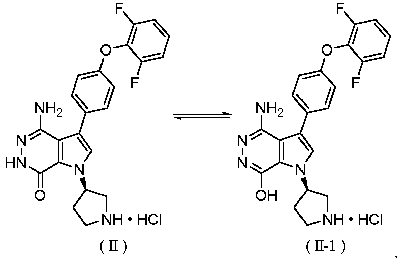

In some embodiments, the carbonyl in the compound of formula (III) can also undergo enol interconversion, specifically as shown below

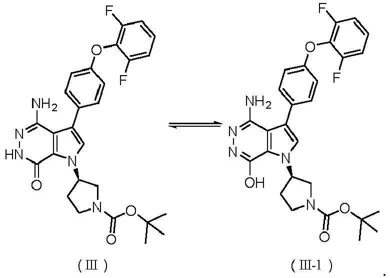

In the above embodiments, the process for preparing the compound of formula (I) from the compound of formula (a) (simplified as: formula (a)→ formula (I)), although this is shown as a one-step reaction, it can be either a one-step or multi-step reaction, depending on the definitions of the L, Y and the substituents on Y.

The present disclosure also provides a compound of formula (c), a salt thereof or a stereoisomer thereof, 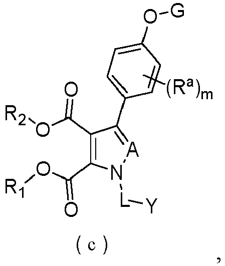 wherein, R^{a}, R₁, R₂, A, G, L, Y and m are as defined in formula (c) above.

In some embodiments, in the above embodiments, the compound of formula (c) is selected from

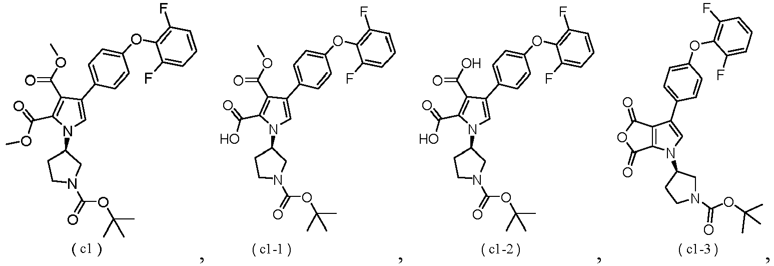

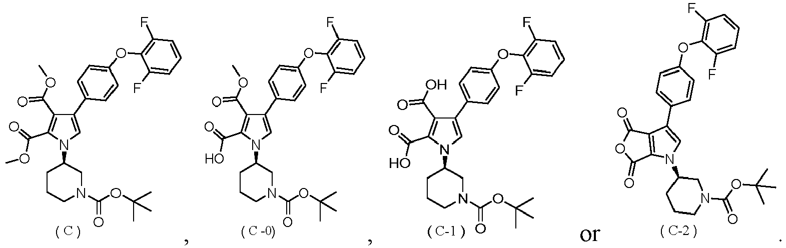

The present disclosure also provides a method for preparing a compound of formula (c) or a stereoisomer thereof, the method comprising 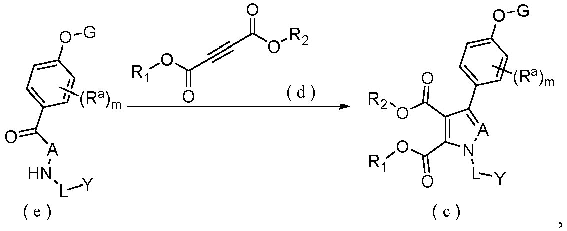 wherein, R^{a}, R₁, R₂, A, G, L, Y and m are as defined in the above formula (c).

In the above embodiments, the method for preparing the compound of formula (c) or the stereoisomer thereof can also comprise 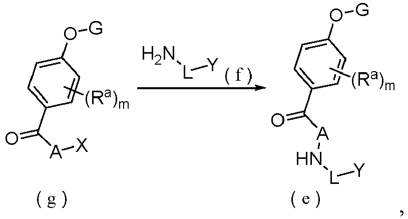 wherein,
R^{a}, A, G, L, Y and m are as defined in formula (c) above; X is selected from fluorine atom, chlorine atom, bromine atom or iodine atom.

In some embodiments, in the formula (g), X is preferably bromine atom.

In the above embodiments, the method for preparing the compound of formula (c) or the stereoisomer thereof can also comprise 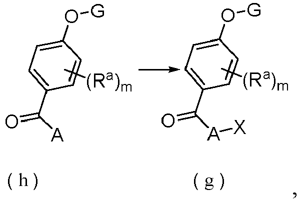 wherein, R^{a}, A, G and m are as defined in the formula (c) above; X is as defined in the formula (g) above.

In the above embodiments, the method for preparing the compound of formula (c) or the stereoisomer thereof can also comprise 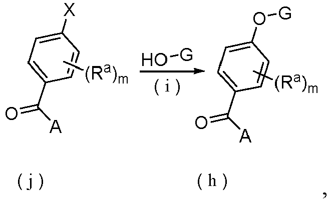 wherein,
R^{a}, A, G and m are as defined in the formula (c) above; X is selected from fluorine atom, chlorine atom, bromine atom or iodine atom.

The present disclosure provides a compound of formula (e), a salt thereof or a stereoisomer thereof 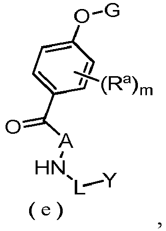 wherein, R^{a}, A, G, L, Y and m are as defined in formula (b).

In some embodiments, Y is selected from optionally substituted pyrrolidinyl or substituted piperidinyl, more preferably

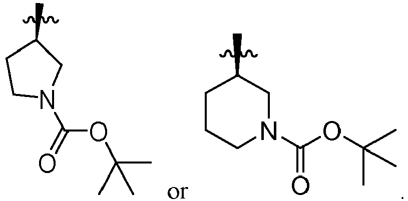

In some embodiments, in the above embodiments, the compound of formula (e) or the salt thereof is selected from

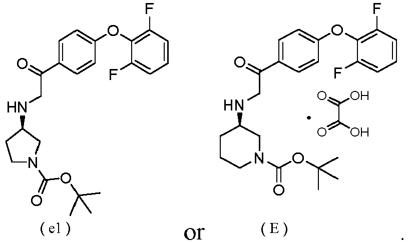

The present disclosure further provides a method for preparing a compound of formula (e) or a stereoisomer thereof, comprising 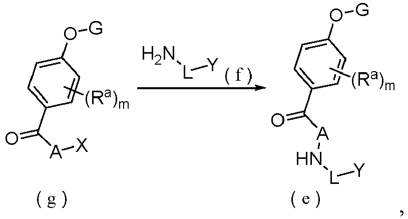 wherein, R^{a}, A, G, L, Y and m are as defined in formula (b); X is selected from fluorine atom, chlorine atom, bromine atom or iodine atom.

In the above embodiments, the method for preparing the compound of formula (e) or the stereoisomer can also comprise 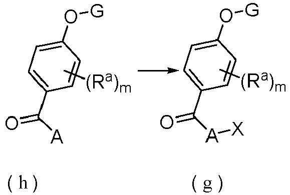 wherein, R^{a}, A, G and m are as defined in formula (b); X is selected from fluorine atom, chlorine atom, bromine atom or iodine atom.

In the above embodiments the method for preparing the compound of formula (e) or the stereoisomer can also comprise 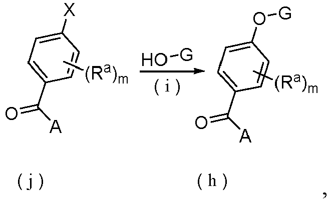 wherein,
R^{a}, A, G and m are as defined in formula (b); X is selected from fluorine atom, chlorine atom, bromine atom or iodine atom.

In some embodiments, X in the formula (j) is preferably fluorine atom.

The present disclosure provides a compound of formula (g), a salt thereof or a stereoisomer thereof 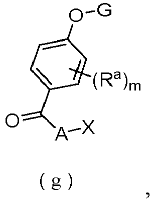 wherein, R^{a}, A, G, X, m are as defined in formula (b).

In some embodiments, in the above embodiments, the compound of formula (g) is selected from

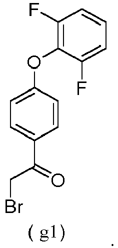

The present disclosure further provides a method for preparing a compound of formula (g) or a stereoisomer thereof, comprising 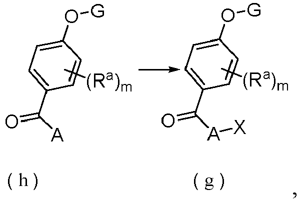 wherein, R^{a}, A, G, X, m are as defined in the formula (g).

In the above embodiments, the method for preparing the compound of formula (g) or the stereoisomer thereof can also comprise 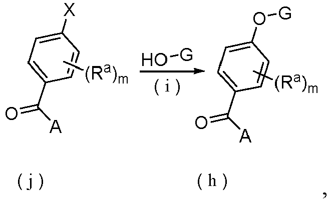 wherein,
R^{a}, A, G and m are as defined in the formula (g); X is selected from fluorine atom, chlorine atom, bromine atom or iodine atom.

In some embodiments, X in the formula (j) is preferably fluorine atom.

The present disclosure also relates to a method for preparing a compound of formula (Ia) or a stereoisomer thereof, comprising

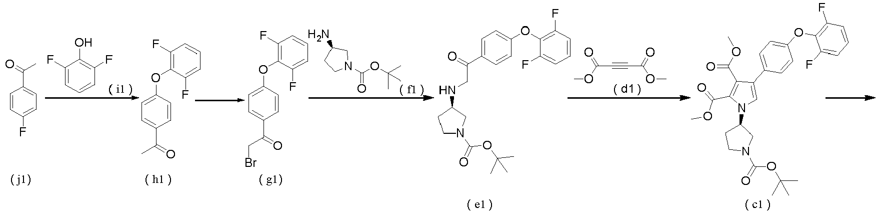

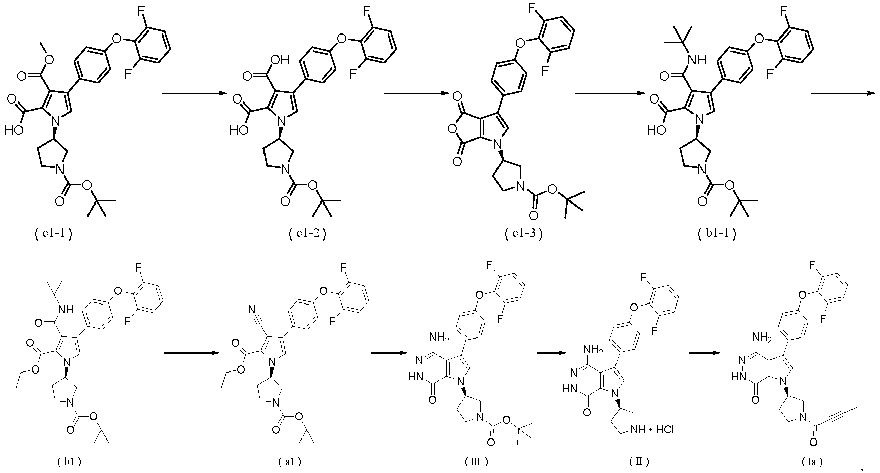

### Step 1, the preparation of the compound of formula (h1)

Carrying out a substitution reaction between the raw materials of the compound of formula (j1) and the compound of formula (i1) in an alkaline medium, adding the reaction mixture to ice water, stirring for crystallization, filtering, and drying to obtain the compound of formula (h1); the alkaline medium is preferably one or more selected from potassium carbonate, sodium carbonate, cesium carbonate, sodium bicarbonate, potassium bicarbonate, sodium hydroxide, potassium hydroxide, lithium hydroxide, sodium acetate, potassium acetate, sodium methoxide, potassium methoxide, triethylamine, *N*,*N-*diisopropylethylamine, ammonia and pyridine, preferably potassium carbonate.

### Step 2, the preparation of the compound of formula (g1)

Dissolving the compound of formula (h1) in a solvent, adding sulfuric acid, adding N-bromosuccinimide, and carrying out a reaction at room temperature, adding the reaction mixture to ice water, stirring for crystallization, filtering, and drying to obtain the compound of formula (g1); the solvent is one or more selected from nitrile solvent, ketone solvent, ether solvent, amide solvent, sulfone solvent and sulfoxide solvent;
the nitrile solvent is preferably one or more selected from acetonitrile, propionitrile and succinonitrile;
the amide solvent is preferably selected from *N*,*N*-dimethylformanmide and/or *N,N-*dimethylacetamide;
the ketone solvent is preferably one or more selected from acetone, butanone and *N-*methylpyrrolidone;
the ether solvent is preferably selected from tetrahydrofuran;
the sulfoxide solvent is preferably selected from dimethyl sulfoxide and/or diethyl sulfoxide;
the sulfone solvent is preferably selected from sulfolane and/or phenylethyl sulfone;
the solvent is preferably nitrile solvent, more preferably acetonitrile.

### Step 3, the preparation of the compound of formula (e1)

Dissolving the compound of formula (f1) and *N,N*-diisopropylethylamine in a solvent, cooling, and adding the solution of the compound of formula (g1) dropwise to carry out a substitution reaction to obtain a crude product of the compound of formula (e1), which is directly used in the next step; the solvent is preferably one or more selected from nitrile solvent, amide solvent, ketone solvent, ether solvent, sulfone solvent and sulfoxide solvent;
the nitrile solvent is preferably one or more selected from acetonitrile, propionitrile and succinonitrile;
the amide solvent is preferably selected from *N*,*N*-dimethylformanmide and/or *N,N-*dimethylacetamide;
the ketone solvent is preferably one or more selected from acetone, butanone and *N-*methylpyrrolidone;
the ether solvent is preferably selected from tetrahydrofuran;
the sulfoxide solvent is preferably selected from dimethyl sulfoxide and/or diethyl sulfoxide;
the sulfone solvent is preferably selected from sulfolane and/or phenylethyl sulfone;
the solvent is preferably an amide solvent, more preferably *N*,*N-*dimethylformanmide.

### Step 4, the preparation of the compound of formula (c1)

Adding the crude compound of formula (e1) to the dimethyl butynedioate compound of formula (d1), which is then dissolved by heating, stirring and carrying out a cyclization reaction to obtain a crude compound of formula (c1), which is directly used in the next step.

### Step 5, the preparation of the compound of formula (c1-1)

Cooling the crude compound of formula (c1), then adding an alkaline solution to the reaction mixture, carrying out a reaction while heating, then adding the reaction mixture to ice water, adding acid to adjust the pH value, crystallizing, filtering, and washing with water, then drying to obtain a crude compound of formula (c1-1), which is used directly in the next step; the acid is preferably one or more selected from hydrochloric acid, hydrofluoric acid, hydrobromic acid, sulfuric acid, phosphoric acid, nitric acid, formic acid, acetic acid, oxalic acid, methanesulfonic acid and *p*-toluenesulfonic acid, preferably hydrochloric acid; the alkali in the alkaline solution is preferably one or more selected from potassium carbonate, sodium carbonate, cesium carbonate, sodium bicarbonate, potassium bicarbonate, sodium hydroxide, potassium hydroxide, lithium hydroxide, sodium acetate, potassium acetate, sodium methoxide, potassium methoxide, triethylamine, *N*,*N*-diisopropylethylamine, ammonia and pyridine, preferably potassium hydroxide.

### Step 6, the preparation of the compound of formula (c1-2)

Dissolving the crude compound of formula (c1-1) in a solvent and alkaline solution, carrying out a reaction while heating, then concentrating, adding the residue to ice water, adding acid to adjust the pH value, crystallizing, filtering, washing with water, then drying to obtain a crude compound of formula (c1-2), which is used directly in the next step; the acid is preferably one or more selected from hydrochloric acid, hydrofluoric acid, hydrobromic acid, sulfuric acid, phosphoric acid, nitric acid, formic acid, acetic acid, oxalic acid, methanesulfonic acid and *p*-toluenesulfonic acid, preferably hydrochloric acid; the alkali in the alkaline solution is preferably one or more selected from potassium carbonate, sodium carbonate, cesium carbonate, sodium bicarbonate, potassium bicarbonate, sodium hydroxide, potassium hydroxide, lithium hydroxide, sodium acetate, potassium acetate, sodium methoxide, potassium methoxide, triethylamine, *N*,*N*-diisopropylethylamine, ammonia and pyridine, preferably potassium hydroxide; the solvent was one or more selected from methanol, ethanol, propanol, butanol, ethylene glycol, acetonitrile, propionitrile, succinonitrile, *N*,*N-*dimethylformanmide, *N*,*N*-dimethylacetamide, acetone, butanone, tetrahydrofuran, dimethyl sulfoxide, sulfolane, *N*-methylpyrrolidone, and acetamide, preferably methanol.

### Step 7, the preparation of the compound of formula (c1-3)

Dissolving the compound of formula (c1-2) in a solvent, adding acetic anhydride, carrying out a reaction while stirring, and concentrating the reaction mixture to obtain the compound of formula (c1-3), which is directly used in the next step; the solvent is one or more preferably selected from halogenated hydrocarbon solvent, aromatic hydrocarbon solvent, ketone solvent, ether solvent, aliphatic hydrocarbon solvent, diol derivative solvent, amide solvent, sulfone solvent and sulfoxide solvent;
the halogenated hydrocarbon solvent is preferably one or more selected from dichloromethane, chloroform and carbon tetrachloride;
the aromatic hydrocarbon solvent is preferably one or more selected from benzene, toluene and xylene;
the ether solvent is preferably one or more selected from ether, ethylene glycol dimethyl ether, tetrahydrofuran and 1,4-dioxane;
the ketone solvent is preferably one or more selected from acetone, butanone and *N-*methylpyrrolidone;
the aliphatic hydrocarbon solvent is preferably selected from nitromethane and/or nitroethane;
the amide solvent is preferably selected from *N*,*N*-dimethylformanmide and/or *N*,*N-*dimethyacetamide;
the sulfoxide solvent is preferably selected from dimethyl sulfoxide and/or diethyl sulfoxide;
the sulfone solvent is preferably selected from sulfolane and/or phenyl ethyl sulfone;
the solvent is preferably ether solvent, more preferably tetrahydrofuran.

### Step 8, the preparation of the compound of formula (b1-1)

Dissolving the residue in a solvent, adding *tert*-butylamine dropwise under an ice water bath, carrying out a reaction while stirring, washing the reaction mixture with water, extracting the aqueous phase and concentrating to obtain a crude compound of formula (b1-1), which is directly used in the next step; the solvent is preferably one or more selected from halogenated hydrocarbon solvent, aromatic hydrocarbon solvent and ether solvent;
the halogenated hydrocarbon solvent is preferably one or more selected from dichloromethane, chloroform and carbon tetrachloride;
the aromatic hydrocarbon solvent is preferably one or more selected from benzene, toluene and xylene;
the ether solvent is preferably selected from ether and/or methyl tertiary butyl ether;
the solvent is preferably halogenated hydrocarbon solvent, more preferably dichloromethane.

### Step 9, the preparation of the compound of formula (b1)

Dissolving the compound of formula (b1-1) in a solvent, adding base, diethyl sulfate or halohydrocarbon, and carrying out a reaction while heating, then cooling and concentrating to obtain a compound of formula (b1); the solvent is preferably one or more selected from halogenated hydrocarbon solvent, aromatic hydrocarbon solvent, ether solvent, ketone solvent, diol derivative solvent, amide solvent, sulfone solvent, sulfoxide solvent and aliphatic hydrocarbon solvent;
the halogenated hydrocarbon solvent is preferably one or more selected from dichloromethane, chloroform and carbon tetrachloride;
the aromatic hydrocarbon solvent is preferably one or more selected from benzene, toluene and xylene;
the ether solvent is preferably one or more selected from ether, ethylene glycol dimethyl ether, tetrahydrofuran and 1,4-dioxane;
the ketone solvent is preferably one or more selected from acetone, butanone and *N-*methylpyrrolidone;
the aliphatic hydrocarbon solvent is preferably selected from nitromethane and/or nitroethane;
the amide solvent is preferably selected from *N*,*N*-dimethylformanmide and/or *N,N-*dimethyacetamide;
the sulfoxide solvent is preferably selected from dimethyl sulfoxide and/or diethyl sulfoxide;
the sulfone solvent is preferably selected from sulfolane and/or phenyl ethyl sulfone;
the solvent is preferably ether solvent, more preferably tetrahydrofuran;
the base is preferably one or more selected from potassium carbonate, sodium carbonate, cesium carbonate, sodium bicarbonate, potassium bicarbonate, sodium hydroxide, potassium hydroxide, lithium hydroxide, sodium acetate, potassium acetate, sodium methoxide, potassium methoxide, triethylamine, *N*,*N*-diisopropylethylamine, ammonia and pyridine, preferably potassium carbonate;
the halohydrocarbon is preferably selected from iodoethane and/or bromoethane.

### Step 10, the preparation of the compound of formula (a1)

Adding the crude compound of formula (b1) to a solvent, adding the solution of trifluoroacetic anhydride slowly thereto at 0°C, carrying out a reaction at room temperature while stirring then quenching, washing with water, extracting the aqueous phase, and concentrating to obtain a crude compound of formula (a1), which is used directly in the next step; the solvent is preferably one or more selected from halogenated hydrocarbon solvent, aromatic hydrocarbon solvent and ether solvent;
the halogenated hydrocarbon solvent is preferably one or more selected from dichloromethane, chloroform and carbon tetrachloride;
the aromatic hydrocarbon solvent is preferably one or more selected from benzene, toluene and xylene;
the ether solvent is preferably selected from ether and/or methyl tertiary butyl ether;
the solvent is preferably halogenated hydrocarbon solvent, more preferably dichloromethane.

### Step 11, the preparation of the compound of formula (III)

Heating the compound of formula (a1) and dissolving in an organic solvent, adding 85% hydrazine hydrate, heating to reflux and reacting, then cooling and concentrating, adding purified water and dichloromethane, then extracting the mixture, combining the organic phases, drying, filtering, washing, and concentrating to obtain the compound of formula (III); the organic solvent is preferably one or more selected from alcohol solvent, ether solvent, ketone solvent, sulfone solvent, sulfoxide solvent, amide solvent and nitrile solvent;
the amide solvent is preferably selected from *N*,*N*-dimethylformanmide and/or *N,N-*dimethyacetamide;
the alcohol solvent is preferably one or more selected from methanol, ethanol, isopropanol and *n*-pentanol;
the ether solvent is preferably selected from tetrahydrofuran and/or 1,4-dioxane;
the ketone solvent is preferably selected from *N*-methylpyrrolidone;
the nitrile solvent is preferably selected from acetonitrile and/or propionitrile;
the organic solvent is preferably one or more selected from acetone, tetrahydrofuran, acetonitrile, *N*-methylpyrrolidone, methanol, ethanol and isopropanol, more preferably ethanol.

### Step 12, the preparation of the compound of formula (II)

Adding organic solvent to the reaction kettle, removing the Boc protecting group under an acidic system, adding the compound of formula (III) while stirring, stirring and reacting at room temperature, concentrating, and drying to obtain the compound of formula (II); the organic solvent is preferably one or more selected from halogenated hydrocarbon solvent, ester solvent, ether solvent and alcohol solvent; the acid in the acidic system is preferably one or more selected from sulfuric acid, hydrochloric acid, acetic acid and trifluoroacetic acid solvent;
the halogenated hydrocarbon solvent is preferably one or more selected from dichloromethane, chloroform and carbon tetrachloride;
the ester solvent is preferably one or more selected from ethyl acetate, dimethyl phthalate and butyl acetate;
the ether solvent is preferably one or more selected from tetrahydrofuran, ether and dioxane;
the alcohol solvent is preferably selected from methanol and/or ethanol;
the organic solvent is preferably one or more selected from dichloromethane, ethyl acetate, tetrahydrofuran and ethanol, more preferably ethyl acetate and/or ethanol.

### Step 13, the preparation of the compound of formula (Ia)

Carrying out a condensation reaction between the compound of formula (II) and 2-butynoic acid under the condition of a condensing agent, adding purified water to the reaction mixture, stirring, and extracting the aqueous phase, washing the organic phase with purified water, drying, filtering, washing, and concentrating the filtrate to obtain the compound of formula (Ia); the condensing agent is preferably one or more selected from carbonyl diimidazole, 1-(3-dimethylaminopropyl)-3-ethylcarbodiimide hydrochloride/1-hydroxyl benzotriazole, 2-(7-benzotriazole oxide)-*N*,*N*,*N'*,*N*'-tetramethylurea hexafluorophosphate, dicyclohexylcarbodiimide/4-*N*,*N*-lutidine, 1-(3-dimethylaminopropyl)-3-ethylcarbodiimide hydrochloride and oxalyl chloride; preferably carbonyl diimidazole and/or 1-(3-dimethylaminopropyl)-3-ethylcarbodiimide hydrochloride.

In some embodiments, the method for preparing the compound of formula (Ia) or the stereoisomer thereof above can also comprise

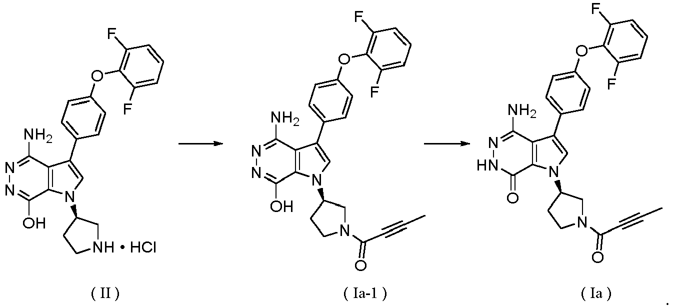

In some embodiments, when L is absent, Y is substituted pyrrolidinyl, and the substituent on Y is *t*-butyloxycarboryl, then the structure is as shown in formula (III), and the compound of formula (Ia) is prepared from the compound of formula (a1) (simplified as: formula (a1) → formula (III) → formula (II) → formula of (Ia)), which is a three-step reaction and is shown as below

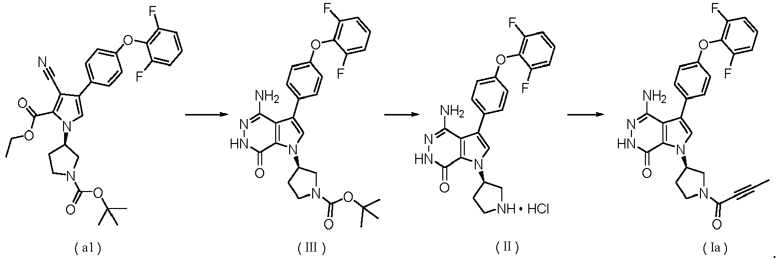

The present disclosure further relates to a method for preparing a compound of formula (a1) or a stereoisomer thereof, comprising

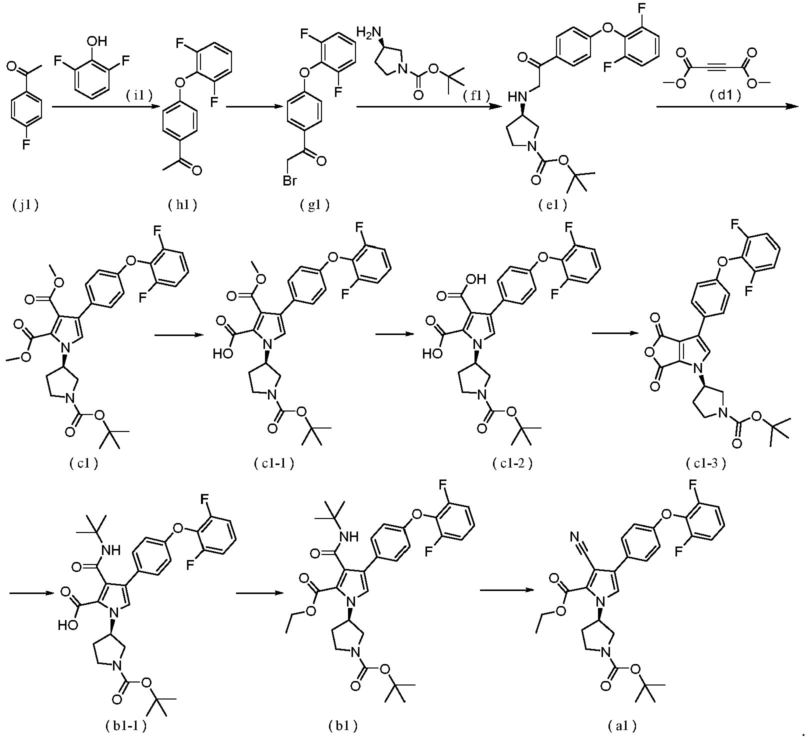

The present disclosure further relates to a method for preparing a compound of formula (c1) or a stereoisomer thereof, comprising

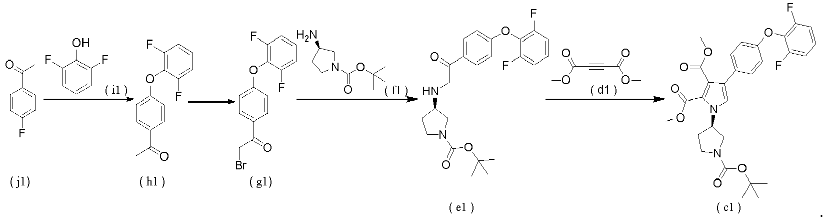

The present disclosure further relates to a method for preparing a compound of formula (IA) or a stereoisomer thereof, comprising

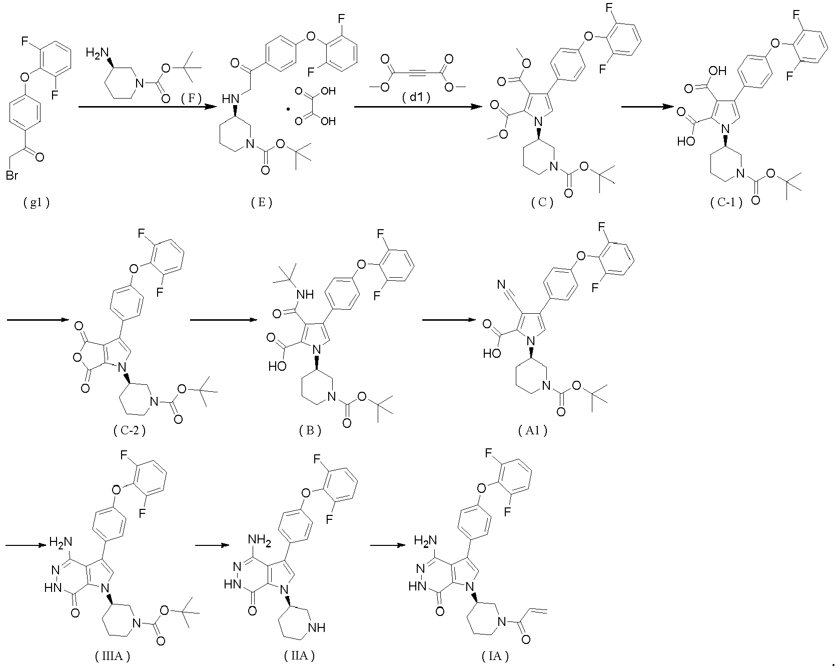

In some embodiments, the method for preparing the compound of formula (IA) or the stereoisomer thereof above can also comprise

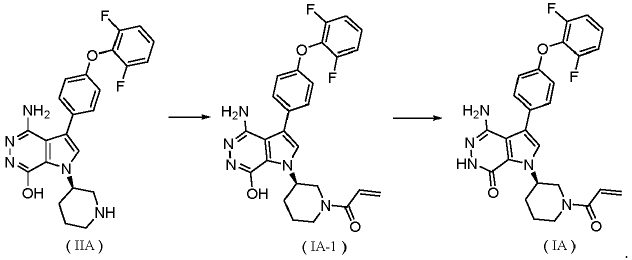

In some embodiments, when L is absent, Y is substituted piperidinyl, and the substitutent on Y is *t*-butyloxycarboryl, then the structure is as shown in formula (IIIA), the compound of formula (IA) is prepared from the compound of formula (A1) (simplified as : formula (A1)→ formula (IIIA) → formula (IIA) → formula (IA)), which is a three-step reaction and is shown as below

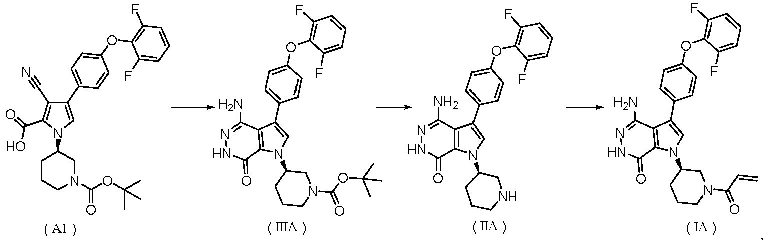

The present disclosure further relates to a method for preparing a compound of formula (A1) or a stereoisomer thereof, comprising

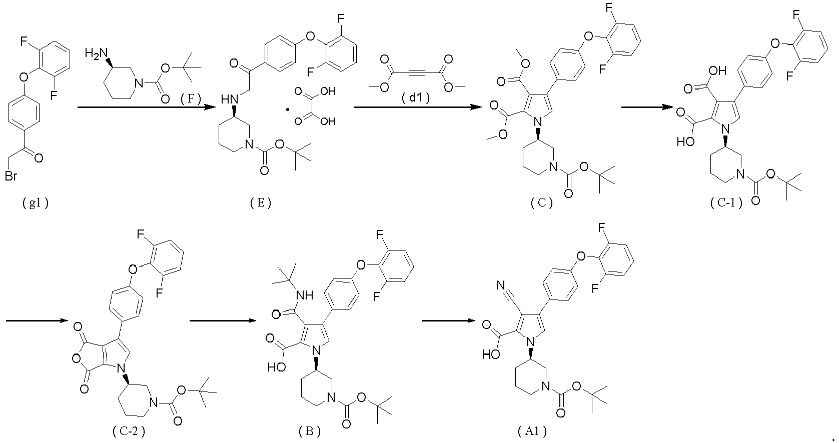

The present disclosure further relates to a method for preparing a compound of formula (C) or a stereoisomer thereof, comprising

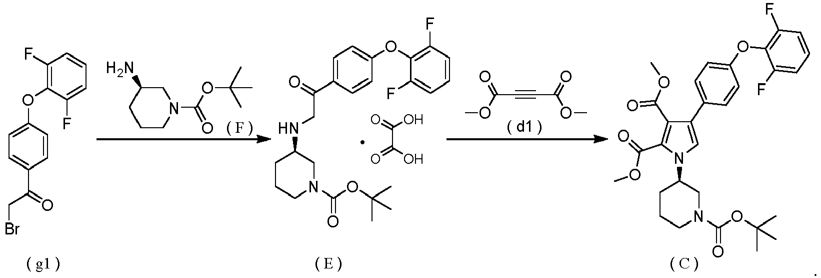

The present disclosure further relates to a method for preparing a compound of formula (Ib) or a stereoisomer thereof, comprising 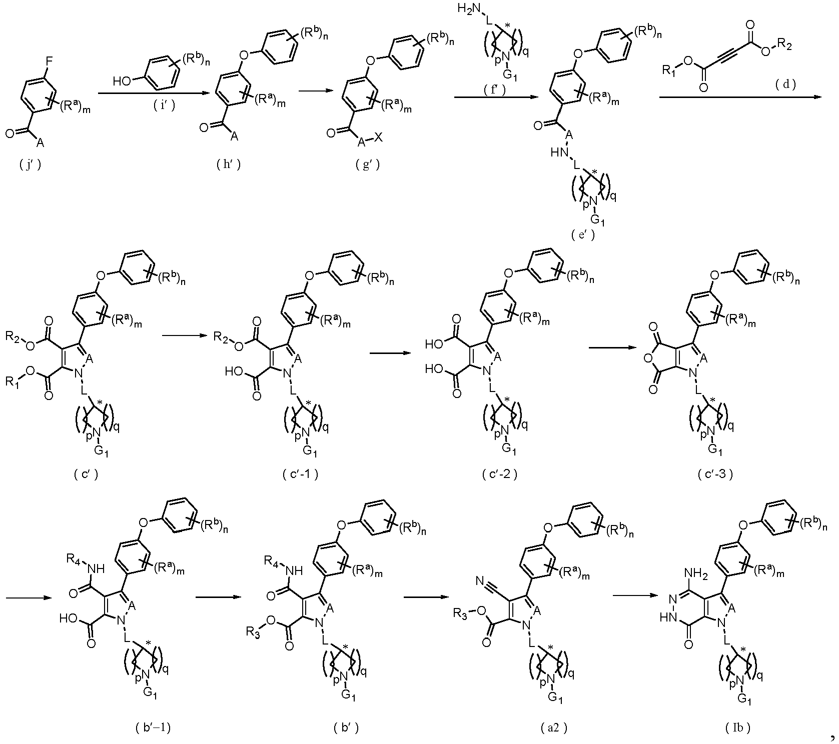 wherein,
A is selected from CR⁰ or N;
R⁰ is selected from hydrogen atom, cyano, carboxyl, hydroxyl, amino, halogen or alkyl;
each of R^{a}, R^{b} is independently selected from hydrogen atom, halogen, hydroxyl, nitro, cyano, carboxyl, amino, alkyl, haloalkyl, haloalkoxyl or alkoxyl;
each of R₁, R₂ is independently selected from alkyl, haloalkyl, benzyl, allyl, trimethylsilyl, triethylsilyl, tetrahydropyranyl or fluorene methyl;
each of R₃, R₄ is independently selected from hydrogen atom, alkyl, alkylcarbonyl, alkoxylcarbonyl, alkylaminocarbonyl, alkylsulfonyl, cycloalkyl, heterocyclyl, aryl or heteroaryl;
L is selected from alkylene or absent;
X is selected from fluorine atom, chlorine atom, bromine atom or iodine atom;
G₁ is selected from hydrogen atom, halogen, hydroxyl, nitro, cyano, carboxyl, amino, alkyl, alkoxyl, alkylamino, dialkylamino, alkylcarbonyl, aldehyde alkyl, alkoxylcarbonyl,aldehyde alkoxyl, alkylaminocarbonyl, alkylcarbonylamino, alkylsulfonyl, alkenyl, alkenylcarbonyl, alkynyl or alkynylcarbonyl;
m=0, 1, 2 or 3;
n=0, 1, 2 or 3;
p=1, 2 or 3;
q=0, 1 or 2;
the carbons marked with * are chiral carbons in S configuration or R configuration.

In some embodiments, in the method for preparing the compound of formula (Ib) or the stereoisomer thereof, the A is preferably CR⁰.

In some embodiments, in the method for preparing the compound of formula (Ib) or the stereoisomer thereof, the R⁰ is preferably hydrogen atom.

In some embodiments, in the method for preparing the compound of formula (Ib) or the stereoisomer thereof, the R^{a} is preferably hydrogen atom.

In some embodiments, in the method for preparing the compound of formula (Ib) or the stereoisomer thereof, the R^{b} is preferably halogen.

In some embodiments, in the method for preparing the compound of formula (Ib) or the stereoisomer thereof, the R₃ is preferably alkyl.

In some embodiments, in the method for preparing the compound of formula (Ib) or the stereoisomer thereof, the R₄ is preferably alkyl.

In some embodiments, in the method for preparing the compound of formula (Ib) or the stereoisomer thereof, each of R₁, R₂ is independently preferably alkyl.

In some embodiments, in the method for preparing the compound of formula (Ib) or the stereoisomer thereof, the L is preferably absent.

In some embodiments, in the method for preparing the compound of formula (Ib) or the stereoisomer thereof, the X is preferably bromine atom.

In some embodiments, in the method for preparing the compound of formula (Ib) or the stereoisomer thereof, the G₁ is preferably alkoxylcarbonyl.

In some embodiments, in the method for preparing the compound of formula (Ib) or the stereoisomer thereof, the n=2.

In some embodiments, in the method for preparing the compound of formula (Ib) or the stereoisomer thereof, the p=2 or 3.

In some embodiments, in the method for preparing the compound of formula (Ib) or the stereoisomer thereof, the q=1.

In some embodiments, in the method for preparing the compound of formula (Ib) or the stereoisomer thereof, the carbon marked with * is R configuration chiral carbon.

In some embodiments, the method for preparing the compound of formula (Ib) or the stereoisomer thereof above can also comprise 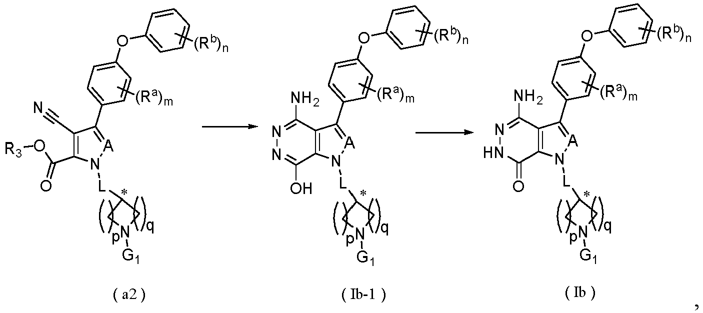 wherein, A, R^{a}, R^{b}, R₃, L, G₁, m, n, p, q and * are as defined above; preferably, G₁ in formula (a2) and formula (Ib-1) are the same, and G₁ in formula (a2) and formula (Ib) are different; G₁ in formula (a2) and formula (Ib-1) is preferably alkoxylcarbonyl; G₁ in formula (Ib-1) is preferably alkenylcarbonyl or alkynylcarbonyl.

The present disclosure further relates to a method for preparing a compound of formula (a2) or a stereoisomer thereof, comprising 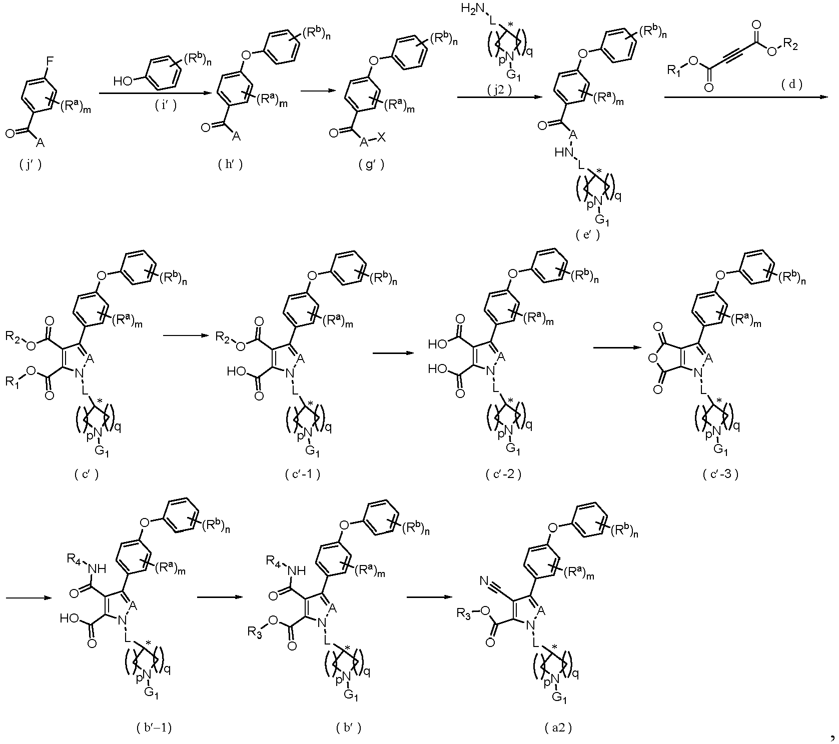 wherein, R^{a}, R^{b}, R₁, R₂, R₃, R₄, A, L, X, G₁, p, q, m, n and * are as defined above.

The present disclosure further relates to a method for preparing a compound of formula (Ic) or a stereoisomer thereof, comprising 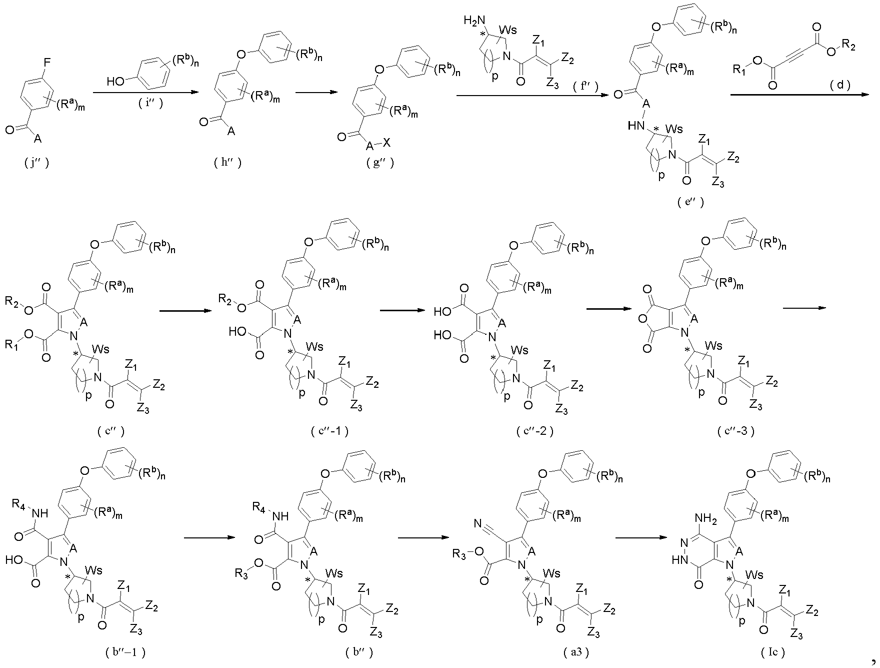 wherein,
A is selected from CR⁰ or N;
R⁰ is selected from hydrogen atom, cyano, carboxyl, hydroxyl, amino, halogen or alkyl;
each of R^{a}, R^{b} is independently selected from hydrogen atom, halogen, hydroxyl, nitro, cyano, carboxyl, amino, alkyl, haloalkyl, haloalkoxyl or alkoxyl;
each of R₁, R₂ is independently selected from alkyl, haloalkyl, benzyl, allyl, trimethylsilyl, triethylsilyl, tetrahydropyranyl or fluorene methyl;
each of R₃, R₄ is independently selected from hydrogen atom, alkyl, alkylcarbonyl, alkoxylcarbonyl, alkylaminocarbonyl, alkylsulfonyl, cycloalkyl, heterocyclyl, aryl or heteroaryl;
Ws is selected from hydrogen atom, halogen, cyano, hydroxyl, alkyl or alkoxyl;
X is selected from fluorine atom, chlorine atom, bromine atom or iodine atom;
each of Z₁, Z₂, Z₃ is independently selected from hydrogen atom, halogen, cyano, hydroxyl, amino, carboxyl, alkyl, alkoxyl, cycloalkyl, heterocyclyl, alkylcarbonyl, aldehyde alkyl, alkoxylcarbonyl, aldehyde alkoxyl, alkylaminocarbonyl, aldehyde alkylamino or alkylsulfonyl, and, Z₁ and Z₂ can form a bond or form a 5-12 memebred cycloalkyl or 5-12 membered heterocyclyl combine with the atoms they are attached to;
m=0, 1, 2 or 3;
n=0, 1, 2 or 3;
p=1, 2 or 3;
the carbons marked with * are chiral carbons in S configuration or R configuration.

In some embodiments, in the method for preparing the compound of formula (Ic) or the stereoisomer thereof, the A is preferably CR⁰.

In some embodiments, in the method for preparing the compound of formula (Ic) or the stereoisomer thereof, the R⁰ is preferably hydrogen atom.

In some embodiments, in the method for preparing the compound of formula (Ic) or the stereoisomer thereof, the R^{a} is preferably hydrogen atom.

In some embodiments, in the method for preparing the compound of formula (Ic) or the stereoisomer thereof, the R^{b} is preferably halogen.

In some embodiments, in the method for preparing the compound of formula (Ic) or the stereoisomer thereof, the R₃ is preferably alkyl.

In some embodiments, in the method for preparing the compound of formula (Ic) or the stereoisomer thereof, the R₄ is preferably alkyl.

In some embodiments, in the method for preparing the compound of formula (Ic) or the stereoisomer thereof, each of R₁, R₂ is independently selected from alkyl.

In some embodiments, in the method for preparing the compound of formula (Ic) or the stereoisomer thereof, the Ws are preferably hydrogen atom.

In some embodiments, in the method for preparing the compound of formula (Ic) or the stereoisomer thereof, the X is preferably bromine atom.

In some embodiments, in the method for preparing the compound of formula (Ic) or the stereoisomer thereof, the Z₁, Z₂, Z₃ is preferably hydrogen atom.

In some embodiments, in the method for preparing the compound of formula (Ic) or the stereoisomer thereof, the n=2.

In some embodiments, in the method for preparing the compound of formula (Ic) or the stereoisomer thereof, the p=2 or 3.

In some embodiments, in the method for preparing the compound of formula (Ic) or the stereoisomer thereof, the carbon marked with * is R configuration chiral carbon.

In some embodiments, the method for preparing the compound of formula (Ic) or the stereoisomers thereof above can also comprise 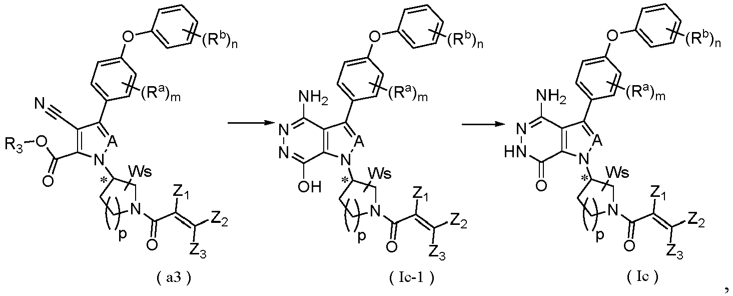 wherein, R^{a}, R^{b}, R₃, A, Ws, Z₁, Z₂, Z₃, p, m, n and * are as defined above.

The present disclosure further relates to a method for preparing a compound of formula (a3) or a stereoisomer thereof, comprising 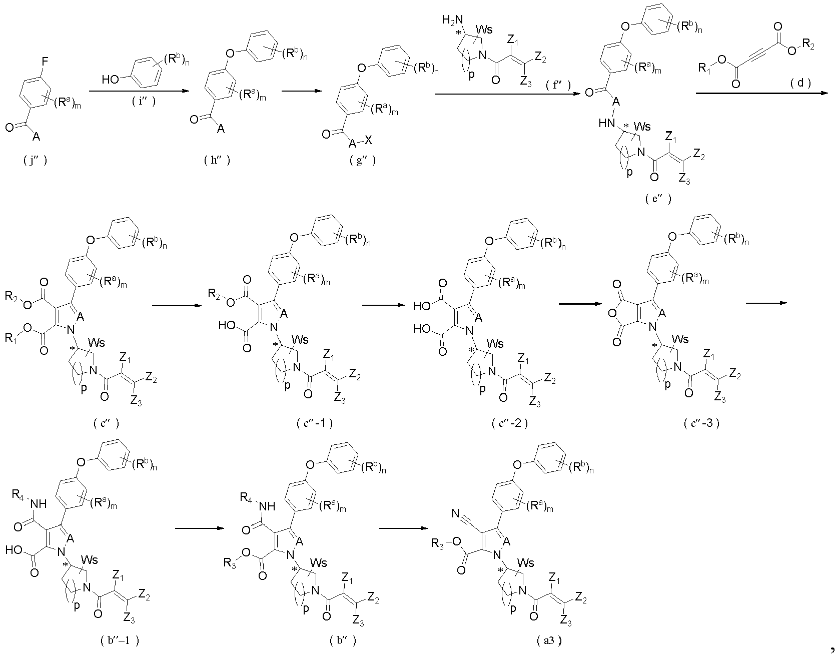 wherein, R^{a}, R^{b}, R₁, R₂, R₃, R₄, A, X, Ws, Z₁, Z₂, Z₃, p, m, n and * are as defined above.

The present disclosure further provides a step for preparing a pharmaceutically acceptable salt of the compound of formula (Ia) by reacting the compound of formula (Ia) with an acid, the acid is preferably selected from an organic acid or inorganic acid, preferably an organic acid; the organic acid is preferably selected from acetic acid, trifluoroacetic acid, oxalic acid, tartaric acid, maleic acid, fumaric acid, *p*-toluenesulfonic acid, benzenesulfonic acid, ethanesulfonic acid or methanesulfonic acid; the inorganic acid is preferably selected from hydrochloric acid, sulfuric acid or phosphoric acid.

### Detailed description of the present disclosure

In order to make it easier to understand the present disclosure, certain technical and scientific terms are specifically defined below. Unless it is obvious that there is a clear definition elsewhere in the present disclosure, otherwise all other technical and scientific terms used herein have the general meaning that is usually understood by a skilled person in the field of the present disclosure.

In the present disclosure, the number of R⁰ in "CR⁰" complements the compound valence of the C atom, so that the C atom is in a saturated valence.

In the present disclosure, when the undefined "N" has an unsaturated valence, it should be considered that the N atom is connected with hydrogen to make the valence of the N atom saturated and formed a stable structure. For example, when the nitrogen-containing heterocycle is opened, and the valence state of the N atom is not saturated, then it should be considered that the N atom is connected with hydrogen to make the valence state of the N atom saturated.

In the present disclosure, the "substituted" refers to one or more hydrogen atoms in the group, preferably at most 5, more preferably 1 to 3 hydrogen atoms (for example, 2) are independently substituted by a corresponding number of substituents.

The "halogen or halogen atom" in the present disclosure refers to fluorine atom, chlorine atom, bromine atom, iodine atom, etc.

The "alkyl" in the present disclosure refers to a linear or branched alkyl containing 1-20 carbon atoms, including, for example, "C₁₋₆ alkyl", "C₁₋₄ alkyl", etc., specific examples include but are not limited to: methyl, ethyl, *n*-propyl, isopropyl, *n*-butyl, isobutyl, *sec*-butyl, *tert*-butyl, *n-*pentyl, isopentyl, 2-methylbutyl, neopentyl, 1-ethylpropyl, *n*-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1,2-dimethylpropyl, etc.

The "alkylene" in the present disclosure refers to the group formed by removing the hydrogen atom from the "alkyl", including, for example, "C₁₋₆ alkylene", "C₁₋₄ alkylene", etc., specific examples include but are not limited to: methylene, ethylene, propylene, isopropylene, butylene, isobutylene, *sec*-butylene, *tert*-butylene, pentylene, isopentylene, neopentylene, *n-*hexylene, isohexylene, etc., the "alkyl" is as defined above.

The "alkenyl" in the present disclosure refers to a linear or branched group containing at least one double bond and a carbon number of 2-20, including, for example, "C₂₋₆ alkenyl, C₂₋₄ alkenyl" and the like. Specific examples include but are not limited to: vinyl, allyl, 2-butenyl, 2-pentenyl, 3-pentenyl, 2-hexenyl, 3-hexenyl, etc.

The "alkynyl" in the present disclosure refers to a linear or branched group containing at least one triple bond and a carbon number of 2-20, including, for example, "C₂₋₆ alkynyl, C₂₋₄ alkynyl" and the like. Specific examples include but are not limited to: ethynyl, propynyl, 2-butynyl, 2-pentnyl, 3-pentnyl, 4-methyl-2-pentnyl, 2-hexynyl, 3-hexynyl, 5-methyl-2-hexynyl, etc.

The "haloalkyl" in the present disclosure refers to a group derived from one or more "halogen atoms" replacing one or more hydrogen atoms on the "alkyl", and the "halogen atoms" and "alkyl" are as defined above.

The "hydroxylalkyl" in the present disclosure refers to a group derived from one or more "hydroxyl" replacing one or more hydrogen atoms on the "alkyl", and the "alkyl" is as defined above.

The "alkoxyl, haloalkoxyl, alkylcarbonyl, aldehyde alkyl, alkoxycarbonyl, aldehyde alkoxyl, alkylcarbonylamino, alkylaminocarbonyl, aldehyde alkylamino, alkylamino, dialkylamino, alkylsulfonylamino, alkylsulfonyl, alkenylcarbonyl or alkynylcarbonyl" in the present disclosure refers to group that connected in the manner of alkyl-O-, haloalkyl-O-, alkyl-C(O)-, H-C(O)-alkyl-, alkyl-O-C(O)-, H-C(O)-alkyl-O-, alkyl-C(O)-NH-, alkyl-NH-C(O)-, H-C(O)-alkyl-NH-, alkyl-NH-, (alkyl)₂-N-, alkyl-S(O)₂-NH-, alkyl-S(O)₂-, alkenyl-C(O)- or alkynyl-C(O)-, wherein "alkyl, haloalkyl, alkenyl, alkynyl" are as defined above.

The "cycloalkyl" in the present disclosure refers to a saturated or partially unsaturated monocyclic or polycyclic cyclic hydrocarbon substituent, which contains 3 to 14 carbon atoms, preferably 3 to 12 carbon atoms or 5 to 12 carbon atoms, more preferably, the cycloalkyl ring contains 3 to 8 carbon atoms, most preferably the cycloalkyl ring contains 5 to 6 carbon atoms, and most preferably cyclopropyl. Non-limiting examples of monocyclic cycloalkyl include cyclopropyl, cyclobutyl, cyclopentyl, cyclopentenyl, cyclohexyl, cyclohexenyl, cyclohexanedienyl, cycloheptyl, cycloheptyltrienyl, cyclooctyl, etc., preferably cyclopropyl, cyclohexenyl. Polycyclic cycloalkyl includes spiro-, fused- and bridge-cycloalkyl.

The "heterocyclyl" in the present disclosure refers to saturated or partially unsaturated monocyclic or polycyclic cyclic hydrocarbon substituent, which contains 3 to 14 ring atoms, of which at least one ring atom is a heteroatom, such as a nitrogen atom, oxygen atom or sulfur atom, the remaining ring atoms are carbon; the ring atoms in the ring structure (such as carbon atoms, nitrogen atoms or sulfur atoms) can be optionally oxidized. It's preferably contains 3 to 12 ring atoms or 5 to 12 ring atoms, wherein 1 to 4 are heteroatoms, more preferably the heterocyclyl ring contains 3 to 8 ring atoms, more preferably the heterocyclyl ring contains 5 to 6 ring atoms. Non-limiting examples of monocyclic heterocyclyl include pyrrolidinyl, piperidinyl, piperazinyl, morpholinyl, thiomorpholinyl, homopiperazinyl, pyranyl, tetrahydrofuran, etc. Polycyclic heterocyclyl includes spiro-, fused- and bridged-heterocyclyl.

The "cyclic acid anhydride" or "cyclic anhydride" in the present disclosure refers to a cyclic structure formed by the dehydration of the dicarboxylic acid in the same organic acid molecule containing O heteroatoms and the C atoms at two adjacent positions of the O heteroatom are oxidized, in which the number of ring atoms is 5 to 8, the common examples are 5-membered and 6-membered cyclic anhydrides, examples of which include but are not limited to:

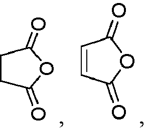

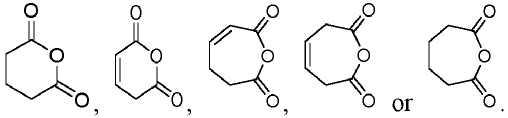

The "aryl" in the present disclosure refers to a 6 to 14-membered all-carbon monocyclic or fused polycyclic (that is, rings sharing adjacent pairs of carbon atoms) group with a conjugated π-electron system, it's preferably 6 to 8-membered, specific examples include but are not limited to phenyl, anthracenyl, phenanthryl, fluorenyl or indenyl.

The "heteroaryl" in the present disclosure refers to a 5- to 15-membered all-carbon monocyclic or fused polycyclic group with a conjugated π-electron system, further comprising 1 to 4 heteroatoms, wherein the heteroatom is one or more selected from oxygen, sulfur or nitrogen. It's preferably 5- to 8-membered heteroaryl, more preferably 5- to 6-membered heteroaryl, specific examples include but not limited to furyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, 2-pyridonyl, 4-pyridonyl, pyrimidinyl, pyridazinyl, pyrazinyl, 1,2,3-triazinyl, 1,3,5-triazinyl, 1,2,4,5-tetrazinyl, azacycloheptyltrienyl, 1,3-diazabicycloheptyltrienyl, azacyclooctatetraenyl, etc.; the heteroaryl can also be fused on an aryl, heterocyclyl or cycloalkyl ring.

The "carbon atom, nitrogen atom or sulfur atom is oxygenated" in the present disclosure refers to the C=O, N=O, S=O or SO₂ structure formed.

The "amide solvent" in the present disclosure refers to a liquid compound in which hydroxyl in the carboxyl of the carboxylic acid molecules is substituted by amino or hydrocarbon amino (-NHR or -NR₂); it can also be regarded as a liquid compound in which the nitrogen atom in ammonia or amine molecule is substituted by acyl; specific examples include but are not limited to: *N*,*N-*dimethylformanmide, *N*,*N-*dimethyacetamide.

The "ester solvent" in the present disclosure refers to a compound with less than 15 carbon atoms formed by the reaction of an organic acid with an alcohol or phenol to lose water, or a low-level ester compound having a functional group of -C(O)O- and a carbon number less than 15, specific examples include but are not limited to: methyl acetate, ethyl acetate, dimethyl phthalate, butyl acetate or propyl acetate.

The "ketone solvent" in the present disclosure refers to a compound in which carbonyl(-C(O)-) is connected to two hydrocarbon groups, according to the different hydrocarbon groups in the molecule, ketone can be divided into aliphatic ketone, alicyclic ketone, aromatic ketone, and saturated ketone and unsaturated ketone, specific examples include but are not limited to: acetone, butanone, acetophenone, methyl isobutyl ketone or methyl pyrrolidone.

The "ether solvent" in the present disclosure refers to a chain compound or a cyclic compound containing an ether bond -O- and a carbon number of 1 to 10, specific examples include but are not limited to: tetrahydrofuran, ether, propylene glycol methyl ether, ethylene glycol dimethyl ether, methyl ter-butyl ether or 1,4-dioxane.

The "alcohol solvent" in the present disclosure refers to a group derived from one or more "hydroxyl" substituting one or more hydrogen atoms on "C₁₋₆ alkyl", the "hydroxyl" and "C₁₋₆ alkyl" are as defined above, specific examples include but are not limited to: methanol, ethanol, isopropanol, *n*-propanol, isoamyl alcohol or trifluoroethanol.

The "nitrile solvent" in the present disclosure refers to a group derived from one or more "cyano" substituting one or more hydrogen atoms on "C₁₋₆ alkyl", the "cyano" and "C₁₋₆ alkyl" are defined above, specific examples include but are not limited to: acetonitrile or propionitrile.

The "halogenated hydrocarbon solvent" in the present disclosure refers to a group derived from one or more "halogen atoms" substituting one or more hydrogen atoms on "C₁₋₆ alkyl", and the "halogen atoms" and "C₁₋₆ alkyl" are as defined above, specific examples include but are not limited to: methyl chloride, dichloromethane, chloroform or carbon tetrachloride.

The "aliphatic hydrocarbon solvent" in the present disclosure refers to a hydrocarbon containing 1-10 carbon atoms with the basic properties of aliphatic compounds, in which the atoms in the molecule are connected to form a chain-like carbon frame, and the two ends of the carbon frame are open and do not form a ring, such as saturated aliphatic hydrocarbon, including alkane solvent, specific examples include but are not limited to: *n*-butane, *n*-pentane, *n*-hexane, *n*-heptane, nitromethane or nitroethane.

The "aromatic hydrocarbon solvent" in the present disclosure refers to a conjugated system having a closed ring in the molecule, and is the general term for carbocyclic compounds and their derivatives with π electron number conforming to Huckel's rule, specific examples include but are not limited to benzene, toluene, cumene or xylene.

The "sulfoxide solvent" in the present disclosure refers to a compound formed by combining a sulfinyl group (-SO-) with a hydrocarbon group, specific examples include but are not limited to: dimethyl sulfoxide, diethyl sulfoxide or benzyl sulfoxide.

The "sulfone solvent" in the present disclosure refers to a compound formed by combining sulfonyl (-S(O)₂-) with a hydrocarbon group, specific examples include but are not limited to: dimethyl sulfone, phenyl ethyl sulfone, diethyl sulfone, diphenyl sulfone or sulfolane.

The "optional" or "optionally" in the present disclosure refers that the event or environment described later can but does not have to occur, and the description includes occasions where the event or environment occurs or does not occur. For example, "heterocyclyl optionally substituted by alkyl" means that an alkyl may but does not have to exist, and the description includes the case where the heterocyclyl is substituted by alkyl and the case where the heterocyclyl is not substituted by alkyl.

### The beneficial effects of the invention

Compared with the prior art, the technical solution of the present disclosure for preparing the compound of formula (I) has the following advantages:
(1) Compared with the prior art, the starting materials and intermediates of the present disclosure are different, providing a completely different synthetic method, and the starting materials and reactants are simple and easy to be purchased.
(2) The yield is improved.
(3) The post-treatment of the reaction is simple, and the crude product can be directly used in the next step reaction, and it is not necessary to purify the product in each step before it can be put into the next step reaction, which is easy for industrial expansion.

### Detailed description of the specific embodiments

The following examples are used to further describe the present disclosure, but these examples do not limit the scope of the present disclosure.

The experimental methods without specific conditions specified in the examples of the present disclosure are usually carried out in accordance with conventional conditions or the conditions suggested by the raw materials or commodities manufacturers. Reagents without specific source are conventional reagents purchased on the market.

The structures of the compounds are determined by nuclear magnetic resonance (NMR) or/and mass spectrometry (MS). NMR shift (δ) is given in the unit of 10⁻⁶ (ppm). NMR is measured by Bruker AVANCE-400 nuclear magnetic instrument, the solvent is heavy water and sodium hydroxide (CDCl₃), and the internal standard is tetramethylsilane (TMS).

Waters Alliance 2695 high-performance chromatograph and Agilent 1200 series liquid chromatograph have been used for HPLC measurement, with octadecyl silane bonded silica as the column packing.

### Example 1

Preparation of (*R*)-4-amino-1-(1-(but-2-ynoyl)pyrrolidin-3-yl)-3-(4-(2,6-difluorophenoxy)phenyl)-1,6-dihydro-7*H*-pyrrolo[2,3-*d*]pyridazin-7-one

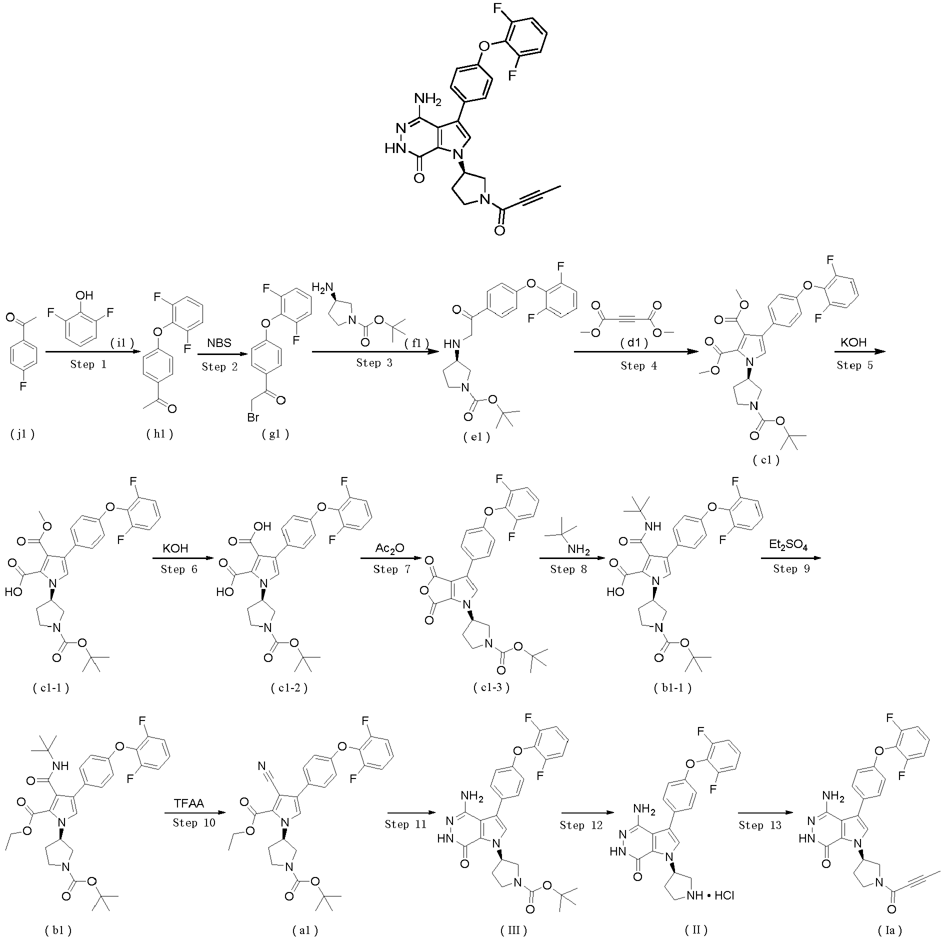

### Step 1, the synthesis of the compound of formula (h1)

The compound of formula (i1) (550 g), the compound of formula (j1), *p-*fluoroacetophenone (700.8 g), potassium carbonate (1.75 kg) and dimethylacetamide (6.4 L) was added into a reaction flask, the temperature was raised to 150°C, and the reaction was stirred for 24 hours. The reaction mixture was poured into ice water (12.5 L), stirred, a solid precipitated, filtered under reduced pressure, and dried to obtain the compound of formula (h1) (960 g) with a yield of 91.5%.

### Step 2, the synthesis of the compound of formula (g1)

The compound of formula (h1) (2 kg) was dissolved in acetonitrile (20 L), sulfuric acid (80 mL) was added, *N*-bromosuccinimide (1.68 kg) was added, after the addition, the reaction was carried out at room temperature for 20 hours overnight, the reaction mixture was poured into ice water (80 L), a solid precipitated out, stirred for 30 minutes and filtered to obtain the crude title product (2.6 kg). The crude product (2.6 kg) was dissolved in methyl tertiary butyl ether (2.6 L), heated to reflux until it was dissolved and clarified, then *n*-hexane (3.4 L) was slowly added, a large number of solids was precipitated when the temperature was cooled down naturally to 40°C, the temperature was kept at 40°C for 30 minutes and then cooled to room temperature. The reaction flask was placed in an ice bath, kept for 2 hours, filtered, and dried to obtain the compound of formula (g1) (2.025 kg) with a yield of 76.8%.

### Step 3, the synthesis of the compound of formula (e1)

The compound of formula (f1) (250.5 g) and *N*,*N*-diisopropylethylamine (197.6g) were dissolved in *N,N*-dimethylformanmide (2700 mL), the mixture was ventilated with argon gas three times, and cooled with an ice-salt bath to -5 to 0°C, the *N*,*N*-dimethylformanmide solution (1300 mL) of the compound of formula (g1) (400 g) was added dropwise, after dripping, the reaction was carried out at -5°C for 3 hours for the next step.

### Step 4, the synthesis of the compound of formula (c1)

The compound of formula (d1) dimethyl butynedioate (521.3 g) was added to the above reaction mixture, the solution was brown and clear after heated to 90°C; the reaction mixture was stirred for 2.5 hours, then the reaction was stopped and used in the next step.

### Step 5, the synthesis of the compound of formula (c1-1)

The system was firstly cooled, then an aqueous solution (2.7 L) of potassium hydroxide (1095 g) was added to the reaction mixture, the reaction was carried out for 5 hours after heated to 95°C, then stopped and cooled down. Ice water (24.0 L) was poured into the above reaction mixture, and concentrated hydrochloric acid was slowly added dropwise under stirring to adjust the pH of the reaction mixture to 4-5, and a large number of solids were precipitated, after stirring for 30 minutes, the mixture was filtered and washed and then dried to obtain the solid for the next step.

### Step 6, the synthesis of the compound of formula (c1-2)

The solid was dissolved in methanol (2.3 L), then an aqueous solution (2.2L) of potassium hydroxide (617.7 g) was added, the mixture was heated to reflux and the reaction was carried out for 6 hours and then stopped; the mixture was concentrated to remove methanol, and the residue was washed into ice water (6.9 L), the pH of the mixture was adjusted to 3-4 using the concentrated hydrochloric acid, then a large number of solids were precipitated, filtered, washed with water until neutral, then the filter cake was collected, and dried to obtain the product (586 g) with a yield of 90.7%.
MS *m*/*z* (LC-MS): 526.56 [M-2].

### Step 7, the synthesis of the compound of formula (c1-3)

The compound of formula (c1-2) (250.0 g) was dissolved in tetrahydrofuran (2.5 L), and acetic anhydride (966 g) was added. After the addition, the reaction was carried out while stirring at room temperature for 10 minutes and refluxed for 4 hours, and then the reaction was stopped, the reaction mixture was concentrated under reduced pressure to obtain the residue, which was used in the next step.
MS *m*/*z* (LC-MS): 533.2 [M+23].

### Step 8, the synthesis of the compound of formula (b1-1)

The residue was dissolved in dichloromethane (2.4 L), *tert*-butylamine (103.8 g) was added dropwise under an ice water bath. After the addition, the reaction was carried out while stirring for 2 hours, and then the reaction was stopped. The reaction mixture was washed with water, separated, and dried over anhydrous sodium sulfate, then filtered, and the organic phase was concentrated to dryness, which was used in the next step.
MS *m*/*z* (LC-MS): 606.2 [M+23].

### Step 9, the synthesis of the compound of formula (b1)

The compound of formula (b1-1) obtained in the previous step was dissolved in tetrahydrofuran (2.76 L), potassium carbonate (130.8 g), diethyl sulfate (109.3 g) were added. The reaction was carried out while stirring and heated to reflux for 10 hours, then the reaction was stopped, the reaction mixture was reduced to room temperature, concentrated to dryness, and purified by column chromatography (ethyl acetate: petroleum ether = 1:5) to obtain the product of the compound of formula (b1), with a yield of 78% and a purity of 97.96%.
MS *m*/*z* (LC-MS): 612.47 [M+1]

### Step 10, the synthesis of the compound of formula (a1)

The compound of formula (b1) obtained in the previous step was added to dichloromethane (1.7 L), trifluoroacetic anhydride (165.7 g) was slowly added in dichloromethane solution (500 mL) at 0°C. After addition, the mixture was slowly raised to room temperature and stirred for 5 hours, and then the reaction was stopped. The reaction was quenched by adding methanol (200 mL), then the reaction mixture was washed with water, dried over anhydrous sodium sulfate, filtered, and the filtrate was concentrated under reduced pressure, the residue was the compound of formula (a1), with a yield of 92 % and a purity of 96.2%.

### Step 11, the synthesis of the compound of formula (III)

Anhydrous ethanol (12.0 kg) was added into the reaction kettle, and the compound of formula (a1) ethyl (*R*)-1-(1-(*t*-butoxycarbonyl)pyrrolidin-3-yl)-3-cyano-4-(4-(2,6-difluorophenoxy)phenyl)-1*H*-pyrrole-2- carboxylate (3.0 kg) was added while stirring, the mixture was heated to 60-65°C to dissolve and clarify, then 85% hydrazine hydrate (9.86 kg) was added. The mixture was heated and refluxed for 9-10 hours, then cooled to below 30°C, concentrated to remove ethanol, and then cooled to below 30°C again, purified water (10 kg) and dichloromethane (15 kg) were added thereto and the phases of the mixture were separated after stirring, the aqueous phase was extracted with dichloromethane (15 kg), the organic phases were combined, dried over anhydrous sodium sulfate (1 kg), filtered, the filter cake was washed with dichloromethane (1 kg), the filtrate was concentrated under reduced pressure to dryness, and purified by silica gel column (dichloromethane : methanol=200:1-80:1) to obtain the product (1.87 kg) with a yield of 64.0%.

### Step 12, the synthesis of the compound of formula (II)

Anhydrous ethanol (12.0 kg) was added to a reaction kettle and the temperature was reduced to 0 to 5°C, hydrogen chloride gas (2.78 kg) was introduced to the reaction kettle, the compound of formula (III) (1.5 kg) was added while stirring, and the temperature of the reaction mixture was controlled between 15 to 25°C, the reaction was carried out for 3-4 hours while stirring. Then the mixture was concentrated to dryness under reduced pressure, and the residue was dried to obtain the product (1.35 kg) with a yield of 100%.

### Step 13, the synthesis of the compound of formula (Ia)

Dichloromethane (39.75 kg) was added into the reaction kettle, the temperature was reduced until the temperature of the reaction mixture was lower than 10°C, then *N,N-*diisopropylethylamine (1.35 kg), the compound of formula (II) (1.5 kg), 2-butynoic acid (438.7 g) and 1-ethyl-(3-dimethylaminopropyl) carbodiimide hydrochloide (1.25 kg) was added successively, after the addition, the reaction was carried out for 3 to 4 hours at 15 to 25°C while stirring. Purified water (20 kg) was then added to the reaction mixture, stirred, the phases were separated and the organic phase was washed with purified water (20 kg), dried over anhydrous sodium sulfate (750 g), filtered, and the filter cake was washed with dichloromethane (1 kg), the filtrate was concentrated to dryness under reduced pressure, and purified by silica gel column (dichloromethane : methanol=150:1-60:1) to obtain the product (1.26 kg) with a yield of 78.9%.
¹H-NMR (400 MHz, CDCl₃) *δ* 11.5 (br, 1H), 7.38-7.40 (d, 2H), 7.16-7.24 (m, 1H), 7.02-7.08 (m, 5H), 6.34-6.38 (m, 1H), 5.30-5.32 (br, 2H), 4.19-4.24 (m, 0.5H), 3.69-3.98 (m, 3.5H), 2.53-2.58 (m, 1H), 2.31-2.37 (m, 1H), 1.96-2.02 (d, 3H).

### Example 2

### Preparation of (R)-1-(1-acryloylpiperidin-3-yl)-4-amino-3-(4-(2,6-difluorophenoxy)phenyl)-1,6-dihydro-7H-pyrrolo[2,3-d]pyridazin-7-one

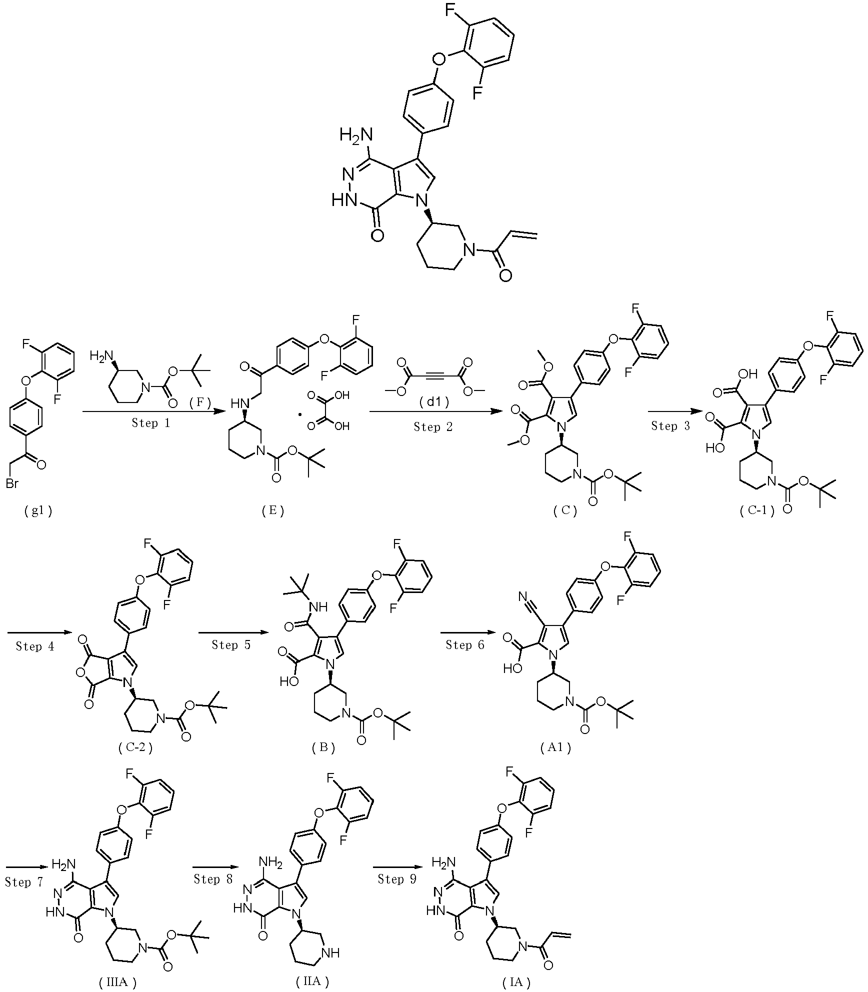

### Step 1, the synthesis of the compound of formula (E)

1-Boc-3-amino piperidine (9.91 g), *N,N*-diisopropylethylamine (7.25 g) were dissolved in *N,N*-dimethylformanmide (100 mL), the mixture was ventilated with argon gas for three times, and cooled to -40 to -45°C with dry ice-acetonitrile, the raw material of a solution of the compound of formula (g1) (14.7 g) in *N,N*-dimethylformanmide (50mL) was added dropwise, after the addition, the reaction was carried out at -45°C for 27.5 hours, then the reaction was stopped, and the reaction mixture was poured into ice water, extracted with dichloromethane (20 mL×3), and the organic phases were combined and dried over anhydrous sodium sulfate, filtered, then oxalic acid (4.45 g) was added to the filtrate, the reaction was stirred at room temperature, and a solid was precipitated, the mixture was concentrated under reduced pressure after 1 hour, then isopropanol (50 mL) was added to the residue, and the reaction was stirred at room temperature for 2 hours, filtered, and the filter cake was collected and dried to obtain the title product (18.9 g) with a yield of 78.4%.

### Step 2, the synthesis of the compound of formula (C)

Methanol (187 mL) was added to the raw material of the compound of formula (E) (18.7 g), the mixture was stirred for 10 minutes, then dimethyl butynedioate (14.9 g) was added, the reaction was refluxed and the solution gradually become clear, which was a brown clear liquid, the reaction mixture was refluxed and stirred for 1.5 hours, the methanol was removed by distillation, then *tert*-butanol was added, and the reaction was carried out for 6.5 hours then stopped, which was used in the next step.

### Step 3, the synthesis of the compound of formula (C-1)

An aqueous solution (200 mL) of potassium hydroxide (34.5 g) was added slowly to the reaction mixture of the compound of formula (C) obtained in the previous step, the reaction was carried out while stirring and under reflux for 28 hours, then stopped, and the reaction mixture was poured into ice water (2 L), acetic acid was slowly added dropwise while stirring, and the pH of the reaction mixture was adjusted to 4 to 5 with acetic acid (60 mL), solids precipitated, after stirring for 30 minutes, the mixture was filtered, and the filter cake was collected, the title product (17.9 g) was obtained after vacuum drying with a yield 94.7%.

### Step 4, the synthesis of the compound of formula (C-2)

The raw material of the compound of formula (C-1) (5.4 g) was dissolved in tetrahydrofuran (54 mL), acetic anhydride (2.04 g) was added thereto, after the addition, the reaction was carried out while stirring at room temperature for 10 minutes and refluxed for an hour, then the reaction was stopped, the reaction mixture was concentrated under reduced pressure to remove the acetic anhydride to obtain the title product, which was used in the next reaction.

### Step 5, the synthesis of the compound of formula (B)

The raw material of the compound of formula (C-2) (5.22 g) was dissolved in dichloromethane (50 mL), *tert*-butylamine (876 mg) was added dropwise, after the addition, the reaction was carried out at room temperature for 2 hours then stopped, and the reaction mixture was washed sequentially with water and saturated sodium chloride solution, the organic phases were combined and dried over anhydrous sodium sulfate, and filtered to obtain the title product, which was used in the next reaction.

### Step 6, the synthesis of the compound of formula (A1)

Under ice bath, a solution of trifluoroacetic anhydride (2.52 g) in dichloromethane solution (5 mL) was slowly added to the dichloromethane solution of the raw material of the compound of formula (B). After the dropwise addition, the reaction was carried out for 5 hours after slowly warmed to room temperature, then stopped, a small amount of methanol was added to the reaction mixture to quench the reaction, and the mixture was washed sequentially with water, saturated sodium bicarbonate solution and saturated sodium chloride solution, the organic phases were combined and dried over anhydrous sodium sulfate, filtered, and the filtrate was concentrated under reduced pressure to obtain the title product (4.9 g) with a yield of 94.0%.

### Step 7, the synthesis of the compound of formula (IIIA)

Under ice bath, the raw material of the compound of formula (A1) (1.04 g) was dissolved in 1,4-dioxane (15 mL), the mixture was stirred for 10 minutes and then *N,N*-carbonyldiimidazole (356.4 mg) was added, after the addition, the reaction was carried out while stirring for 21 hours after the temperature was warmed to room temperature slowly, then stopped; under ice bath, hydrazine hydrate (10 g) was added to the reaction mixture, the reaction was carried out under reflux for 9 hours then stopped, the reaction mixture was extracted with dichloromethane, the organic phase was collected and dried over anhydrous sulfate, filtered, and the filtrate was concentrated under reduced pressure, and purified by silica gel column chromatography (eluent is dichloromethane : methanol=500:1-100:1) to obtain the title product (825 mg) with a yield of 76.8%.

### Step 8, the synthesis of the compound of formula (IIA)

The raw material of the compound of formula (IIIA) (15 g) was added to dichloromethane (300 mL), and trifluoroacetic acid (75 mL) was added thereto, the reaction was carried out for 3 hours then stopped; the reaction mixture was concentrated under reduced pressure and dissolved with dichloromethane (300 mL), saturated sodium bicarbonate solution was added dropwise to adjust the pH to 8 to 9, then the phases were separated, the aqueous phase was extracted with dichloromethane (150 mL×3), the organic phases were combined and dried over anhydrous sodium sulfate, filtered, and the filtrate was distilled under reduced pressure to obtain the title product (10.5 g) with a yield of 86.3%.

### Step 9, the synthesis of the compound of formula (IA)

The compound of formula (IIA) (10.5 g) was dissolved in dichloromethane (250 mL) at 0°C, *N,N*-diisopropylethylamine (10.8 mL) was added thereto, a solution of acrylic chloride (3.1 g) in dichloromethane (50 mL) was added dropwise at 0 to 5°C, the reaction was carried out at 0 to 5°C for 2 hours while stirring then stopped; methanol (5 mL) was added to quench the reaction, and then saturated ammonium chloride solution (100 mL) was added, the phases were separated, and the aqueous phase was extracted with dichloromethane (100 mL×3), the organic phases were combined and distilled under reduced pressure, the residue was purified by column chromatography (eluent: methanol: dichloromethane=1:200-1:100-1:50) to obtain title product (9.55 g) with a yield of 80.9%.
MS *m*/*z* (LC-MS): 492.2 [M+1].
¹H-NMR (400MHz,DMSO-*d*₆) *δ* 11.43 (d, 1H), 7.71 (d, 1H), 7.47 (d, 2H), 7.40-7.33 (m,2H), 7.05 (d, 2H), 6.99-6.84 (m, 1H), 6.12 (d, 1H), 6.64-5.73 (dd, 1H), 5.40 (d, 1H), 4.75 (s, 2H), 4.60-4.35 (m, 1.5H), 4.08 (d, 0.5H), 3.21-3.13 (m, 1H), 2.67 (t, 1H), 2.25-2.13 (m, 2H), 1.86(m, 1H), 1.52(m, 1H).

Although the specific embodiments of the present disclosure are described above, those skilled in the art should understand that these are only examples, and various changes or modifications can be made to these embodiments without departing from the principles and essence of the present disclosure. Therefore, the scope of protection of the present disclosure is defined by the appended claims.

## Claims

1. A compound of formula (b), a salt thereof or a stereoisomer thereof, 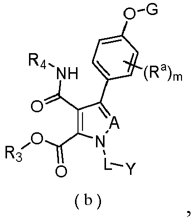 wherein,
A is selected from CR⁰ or N;
R⁰ is selected from hydrogen atom, cyano, carboxyl, hydroxyl, amino, halogen or alkyl;
R^{a} is selected from hydrogen atom, halogen, hydroxyl, nitro, cyano, carboxyl, amino, alkyl, haloalkyl, haloalkoxyl or alkoxyl;
each of R₃, R₄ is independently selected from hydrogen atom, alkyl, alkylcarbonyl, alkoxylcarbonyl, alkylaminocarbonyl, alkylsulfonyl, cycloalkyl, heterocyclyl, aryl or heteroaryl;
G is selected from optionally substituted aryl, heteroaryl, cycloalkyl or heterocyclyl, the substituent is selected from hydrogen atom, halogen, hydroxyl, nitro, cyano, carboxyl, amino, alkyl, alkoxyl, alkylamino, hydroxylalkyl, dialkylamino, alkylcarbonyl, aldehyde alkyl, alkoxycarbonyl, aldehyde alkoxyl, alkylcarbonylamino, alkylaminocarbonyl, alkylsulfonyl, alkenyl, alkenylcarbonyl, alkynyl or alkynylcarbonyl;
L is selected from alkylene or absent;
Y is selected from optionally substituted cycloalkyl, heterocyclyl, aryl or heteroaryl, the substituent is selected from halogen, cyano, alkylcarbonyl, alkoxylcarbonyl, alkylcarbonylamino, alkylsulfonyl, alkylsulfonylamino, alkyl, cycloalkyl, alkenyl, alkenylcarbonyl, alkynyl or alkynylcarbonyl; Y is preferably optionally substituted 3-8 membered heterocyclyl, more preferably optionally substituted pyrrolidinyl or optionally substituted piperidinyl;
m=0, 1, 2 or 3.

2. The compound as defined in claim 1, wherein the compound is selected from

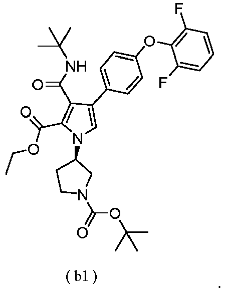

3. A method for preparing a compound of formula (b) or a stereoisomer thereof, wherein the method comprises 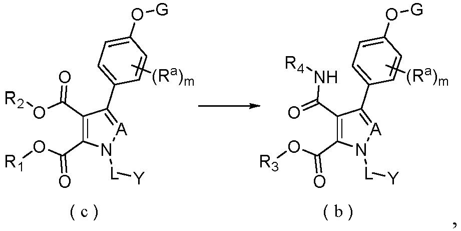 wherein, R^{a}, R₃, R₄, A, G, L, Y and m are as defined in claim 1;
each of R₁, R₂ is independently selected from hydrogen atom, alkyl, haloalkyl, benzyl, allyl, trimethylsilyl, triethylsilyl, tetrahydropyranyl or fluorene methyl, or R₁ and R₂ combine with the groups they are attached to form a 5-membered cyclic anhydride.

4. The method as defined in claim 3, wherein the method further comprises

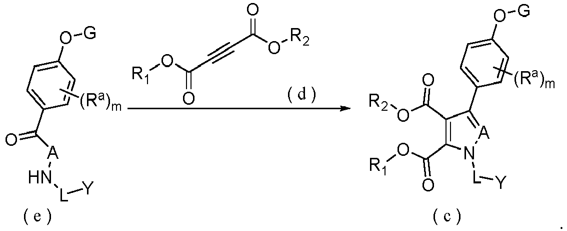

5. The method as defined in claim 4, wherein the method further comprises 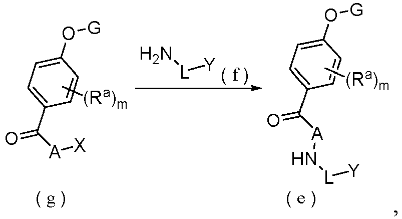 wherein,
X is selected from fluorine atom, chlorine atom, bromine atom or iodine atom.

6. The method as defined in claim 5, wherein the method further comprises

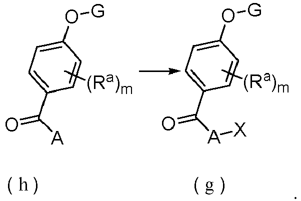

7. The method as defined in claim 6, wherein the method further comprises 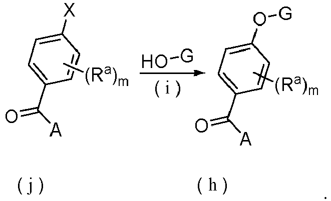 wherein,
X is selected from fluorine atom, chlorine atom, bromine atom or iodine atom.

8. A method for preparing the compound of formula (a) or a stereoisomer thereof, wherein the method comprises 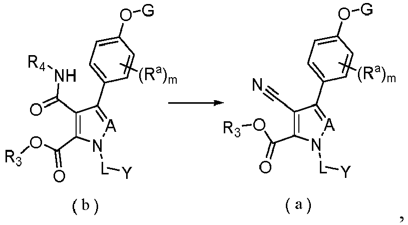 optionally, the method further comprises the method for preparing the compound of formula (b) as defined in any one of claims 3-7;
wherein, R^{a}, R₃, R₄, A, G, L, Y and m are as defined in claim 1.

9. A method for preparing the compound of formula (I) or a stereoisomer thereof, wherein the method comprises 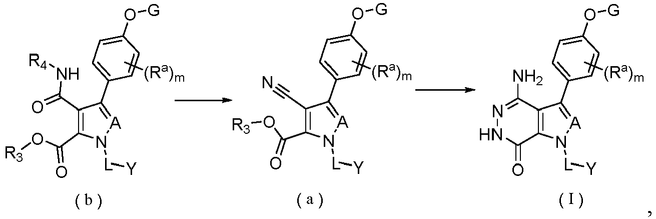 optionally, the method further comprises the method for preparing the compound of formula (b) as defined in any one of claims 3-7;
wherein, R^{a}, R₃, R₄, A, G, L, Y and m are as defined in claim 1.

10. A compound of formula (c), a salt thereof or a stereoisomer thereof, 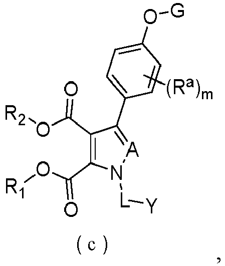 wherein, R^{a}, R₁, R₂, A, G, L, Y and m are as defined in claim 3.

11. The compound as defined in claim 10, wherein the compound is selected from

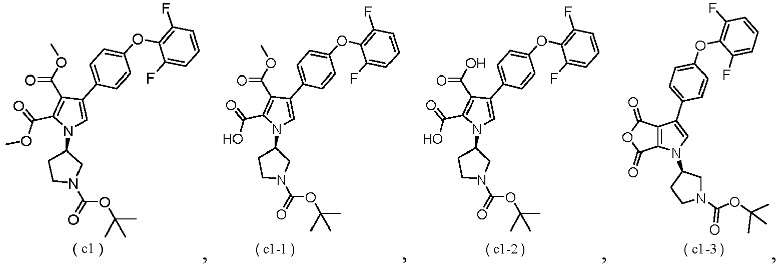

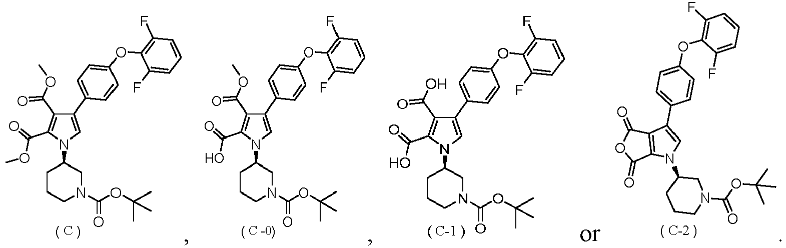

12. A method for preparing a compound of formula (c) or a stereoisomer, wherein the method comprising 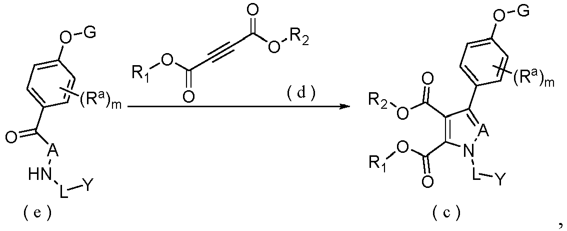 wherein, R^{a}, R₁, R₂, A, G, L, Y and m are as defined in claim 10.

13. The method as defined in claim 12, wherein the method further comprises 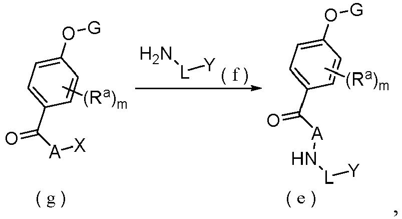 wherein,
X is selected from fluorine atom, chlorine atom, bromine atom or iodine atom.

14. The method as defined in claim 13, wherein the method further comprises

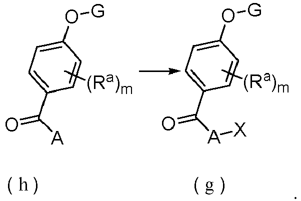

15. The method as defined in claim 14, wherein the method further comprises 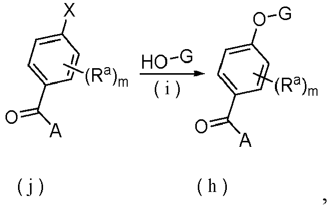 wherein,
X is selected from fluorine atom, chlorine atom, bromine atom or iodine atom.

16. A compound of formula (e), a salt thereof or a stereoisomer thereof 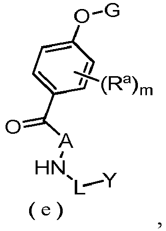 wherein, R^{a}, A, G, L, Y and m are as defined in claim 4.

17. The compound as defined in claim 16, wherein the compound is selected from

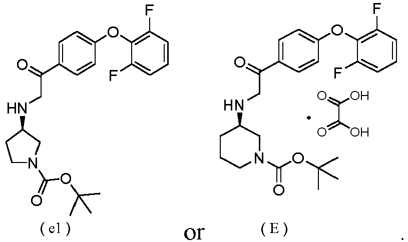

18. A method for preparing a compound of formula (e) or a stereoisomer thereof, wherein the method comprises 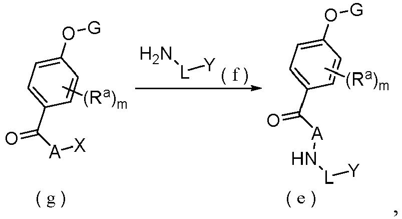 wherein, R^{a}, A, G, L, Y and m are as defined in claim 16;
X is selected from fluorine atom, chlorine atom, bromine atom or iodine atom.

19. The method as defined in claim 18, wherein the method further comprises

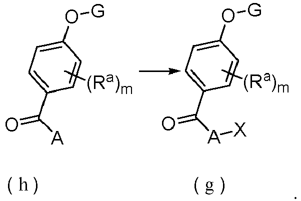

20. The method as defined in claim 19, wherein the method further comprises 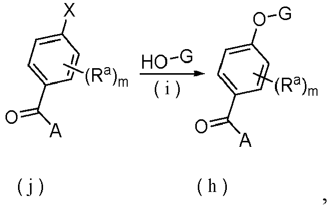 wherein,
X is selected from fluorine atom, chlorine atom, bromine atom or iodine atom.

21. A compound of formula (g), a salt thereof or a stereoisomer thereof 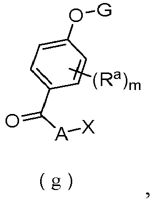 wherein, R^{a}, A, G, X, m are as defined in claim 5.

22. The compound as defined in claim 21, wherein the compound is selected from

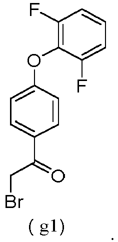

23. A method for preparing a compound of formula (g) or a stereoisomer thereof, wherein the method comprises 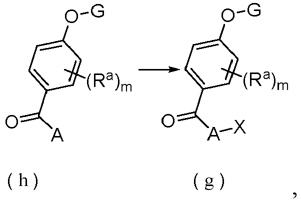 wherein, R^{a}, A, G, X, m are as defined in claim 21.

24. The method as defined in claim 23, wherein the method further comprises 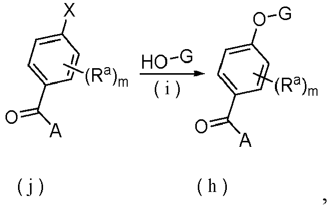 wherein,
X is selected from fluorine atom, chlorine atom, bromine atom or iodine atom.

25. A method for preparing a compound of formula (Ia) or a stereoisomer thereof, wherein the method comprises

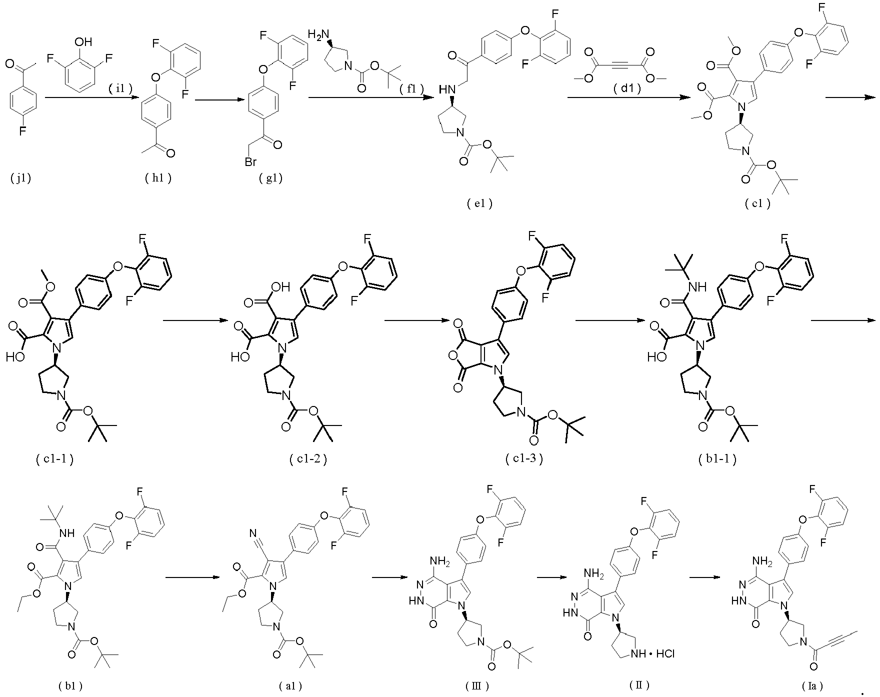

26. A method for preparing a compound of formula (a1) or a stereoisomer thereof, wherein the method comprises

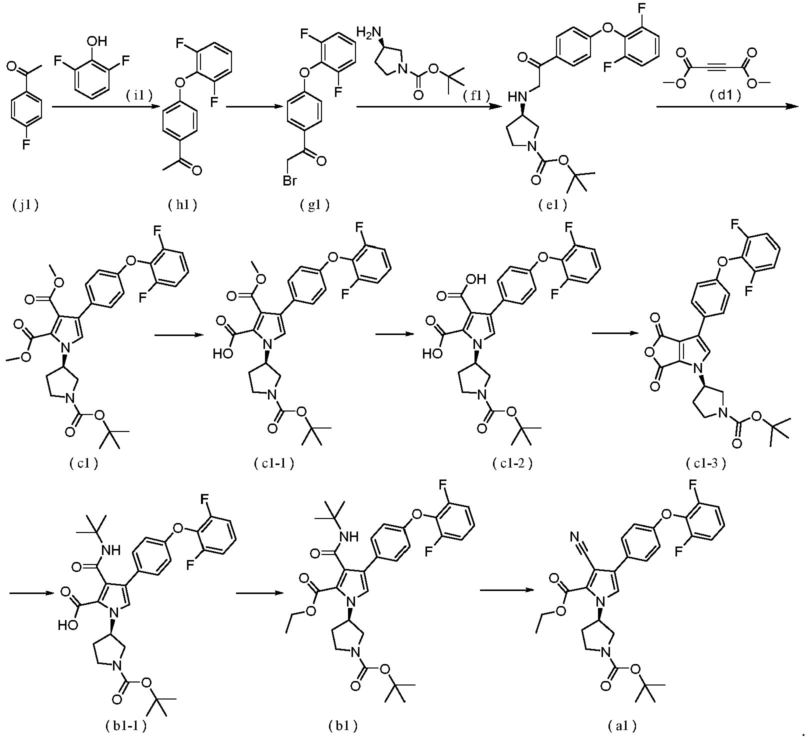

27. A method for preparing a compound of formula (c1) or a stereoisomer thereof, wherein the method comprises

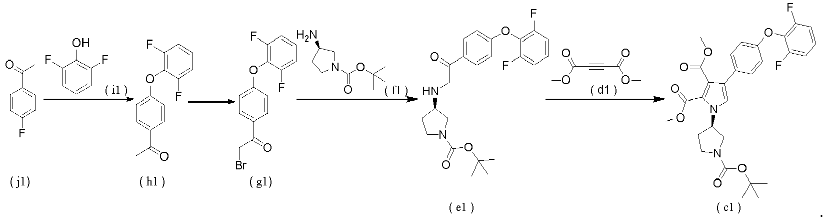

28. A method for preparing a compound of formula (IA) or a stereoisomer thereof, wherein the method comprises

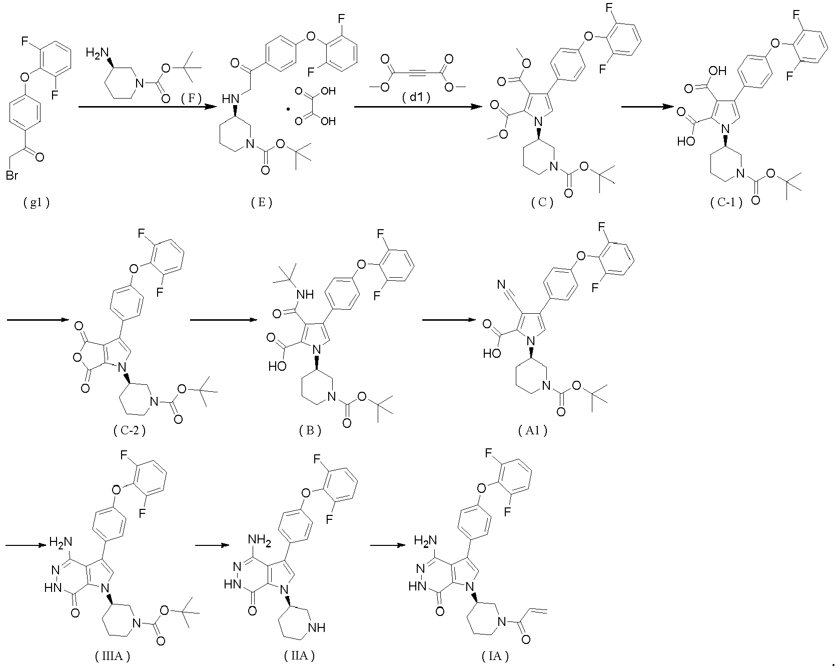

29. A method for preparing a compound of formula (A1) or a stereoisomer thereof, wherein the method comprises

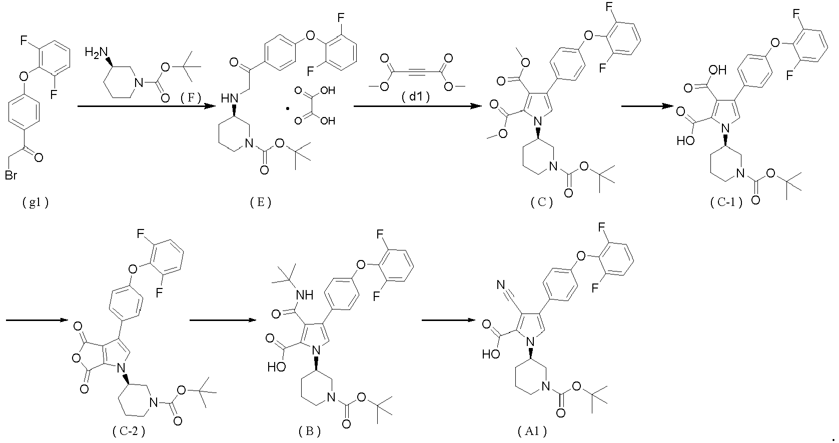

30. A method for preparing a compound of formula (C) or a stereoisomer thereof, wherein the method comprises
